(19) Europäisches Patentamt / European Patent Office / Office européen des brevets

(11) **EP 3 377 598 B1**

(12) **EUROPEAN PATENT SPECIFICATION**

(45) Date of publication and mention
of the grant of the patent:
**10.08.2022   Bulletin 2022/32**

(21) Application number: **16801031.2**

(22) Date of filing: **18.11.2016**

(51) International Patent Classification (IPC):
**C10L 3/10** *(2006.01)*      **C10G 70/06** *(2006.01)*
**C07C 7/11** *(2006.01)*      **B01D 3/40** *(2006.01)*
**B01D 53/14** *(2006.01)*

(52) Cooperative Patent Classification (CPC):
(C-Sets available)
**C10L 3/10; B01D 3/40; B01D 53/1487; C07C 7/11;
C10G 70/06;** B01D 53/1493; B01D 2252/202;
B01D 2252/2021; B01D 2252/2023;
B01D 2252/2025; B01D 2252/20405;
B01D 2252/2041; B01D 2252/20415;
B01D 2252/20442; B01D 2252/20447;      (Cont.)

(86) International application number:
**PCT/GB2016/053602**

(87) International publication number:
**WO 2017/085504 (26.05.2017 Gazette 2017/21)**

(54) **HYDROCARBON SEPARATION PROCESS USING A POLAR SOLVENT**

VERFAHREN ZUR ABTRENNUNG VON KOHLENWASSERSTOFFEN UNTER VERWENDUNG
EINES POLAREN LÖSUNGSMITTELS

PROCÉDÉ DE SÉPARATION D'HYDROCARBURES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **19.11.2015   GB 201520405**

(43) Date of publication of application:
**26.09.2018   Bulletin 2018/39**

(73) Proprietors:
• **Oxford University Innovation Limited
Oxford, Oxfordshire OX2 0JB (GB)**
• **King Abdulaziz City for Science and Technology
Riyadh 11442 (SA)**

(72) Inventors:
• **XIAO, Tiancun
Oxford, Oxfordshire OX1 3QR (GB)**
• **ZHANG, Zhaoxi
Oxford, Oxfordshire OX1 3QR (GB)**
• **EDWARDS, Peter P
Oxford, Oxfordshire OX1 3QR (GB)**
• **AL-MEGREN, Hamid
Riyadh 11442 (SA)**

(74) Representative: **J A Kemp LLP
80 Turnmill Street
London EC1M 5QU (GB)**

(56) References cited:
WO-A1-2015/089446      FR-A1- 2 843 403
US-A- 3 527 837          US-A- 3 770 622
US-A- 5 085 741          US-A- 5 298 156

(52) Cooperative Patent Classification (CPC): (Cont.)
B01D 2252/20452; B01D 2252/20457;
B01D 2252/20468; B01D 2252/20473;
B01D 2252/205; B01D 2252/2056; B01D 2256/24;
B01D 2257/7022; C10L 2290/12; C10L 2290/541;
C10L 2290/543; C10L 2290/544

C-Sets
**C07C 7/11, C07C 9/06;**
**C07C 7/11, C07C 9/08;**
**C07C 7/11, C07C 11/04;**
**C07C 7/11, C07C 11/06**

**Description**

**FIELD OF THE INVENTION**

The invention relates to a process for separating one or more hydrocarbons from a mixture of hydrocarbons, which process is defined in claim 1.

**BACKGROUND TO THE INVENTION**

Mixtures of light olefins and paraffins produced in the petroleum refining process are often used as refinery fuel. In recent years there has been much interest in the recovery of light olefins from these streams, such as the off-gas of crude oil refining and catalytic cracking process (petroleum pyrolysis gas), (Eldridge, R. B. Industrial & engineering chemistry research 1993, 32, 2208) driven largely by the escalating demand for feedstock for production of polyethylene and polypropylene (Ruthven, D. M.; Reyes, S. C. Microporous and mesoporous materials 2007, 104, 59). Ethylene and propylene are the key building block for the production of important petrochemicals, such as polyethylene, polypropylene, acrylonitrile, ethylene oxide, propylene oxide, ethylbenzene, cumene, phenol, isopropylic alcohol and many others ((a) Corma, A.; Melo, F.; Sauvanaud, L.; Ortega, F. Catalysis today 2005, 107, 699(b) Baker, L. M.; Carrick, W. L. The Journal of Organic Chemistry 1970, 35, 774(c) Kilty, P.; Sachtler, W. Catalysis Reviews Science and Engineering 1974, 10, 1(d) Du, Y.; Wang, H.; Chen, S. Journal of Molecular Catalysis A: Chemical 2002, 179, 253). Particularly for the propylene, its global demand during 2006 saw a growing rate estimated at 5.5% bringing total propylene consumptions up to about 69 million metric tons (Ortiz, A.; Ruiz, A.; Gorri, D.; Ortiz, I. Separation and Purification Technology 2008, 63, 311) and the 5-year outlook shows world propylene demand growth to average slightly less than 5% per year (Chemical Market Associates, I. C.; Chemical Market Associates, I. C., Ed. Houston, 2006). The production of polymers and other specialty chemicals from mono-olefins such as ethylene and propylene requires the olefins to be of extremely high purity (>99.9%), and since light olefins are commonly produced together with paraffin hydrocarbons (e.g., ethane and propane), the techniques for separating the both hydrocarbons are of primary importance in the petrochemical industry (Reine, T. A.; Eldridge, R. B. Industrial & engineering chemistry research 2005, 44, 7505).

Environmental regulations such as the Clean Air Act will require reduction of hydrocarbon emissions from chemical processing facilities to low levels, and waste hydrocarbon streams from polyolefin processes and polymer storage facilities, which are typically flared, must be dealt with in an environmentally acceptable manner (Safarik, D. J.; Eldridge, R. B. Industrial & Engineering Chemistry Research 1998, 37, 2571). These regulation requirements also drive the industries and research institutions to develop efficient and sustainable methods to treat the emissions, and then, recover and utilize the light olefins/paraffins from these streams.

However, recovery of olefins in these streams would be a substantial conservation of resources. Because as major and conventional mixture gas separation process, industrial olefins/paraffins separations heavily rely upon energy intensive technologies, which represent a class of the most important and also most costly separations in the chemical and petrochemical industry. It is also extremely challenging due to the physicochemical similarities between those two types of molecules (Safarik, D. J.; Eldridge, R. B. Industrial & Engineering Chemistry Research 1998, 37, 2571; Yang, R.; Kikkinides, E. AIChE Journal 1995, 41, 509; Ortiz, A.; Maria Galán, L.; Gorri, D.; de Haan, A. B.; Ortiz, I. Industrial & Engineering Chemistry Research 2010, 49, 7227; Krull, F.; Medved, M.; Melin, T. Chemical Engineering Science 2007, 62, 5579; Bryan, R. Sep. Purif. ReV. 2004, 33, 157).

As the most traditional separation process, cryogenic distillation has been used for over 60 years for these separations (Keller, G. E.; Marcinkowsky, A. E.; Verma, S. K.; Williamson, K. D. Olefin recovery and purification via silver complexation; Marcel Dekker: New York, 1992). Ethane/ethylene and propane/propylene separations are being performed by these highly energy-intensive distillations. Ethane/ethylene separation is carried out at about 248K and 23bar in a column containing over 100 trays, and propane/propylene separation is process with 150-200 trays at cryogenic temperatures between 233 and 183K and pressures ranging from 16 to 20bar. The reason for the extreme conditions used in such distillations is the similar relative volatilities and boiling points of these olefins and paraffins (Ortiz, A.; Ruiz, A.; Gorri, D.; Ortiz, I. Separation and Purification Technology 2008, 63, 311; Yang, R.; Kikkinides, E. AIChE Journal 1995, 41, 509; Krull, F.; Medved, M.; Melin, T. Chemical Engineering Science 2007, 62, 5579; Keller, G. E.; Marcinkowsky, A. E.; Verma, S. K.; Williamson, K. D. Olefin recovery and purification via silver complexation; Marcel Dekker: New York, 1992).

A 1991 DOE study estimated that 122BTU of energy was used yearly for olefin/paraffin distillation. Even at that time, the capital cost for a world-class ethylene unit had already exceeded 500 million U.S. dollars. The olefin/paraffin separations train contributed a major part of this cost. This large energy and capital investment requirement provides the incentive for ongoing olefin/paraffin separation technology research (Eldridge, R. B. Industrial & engineering chemistry research 1993, 32, 2208).

Olefin/paraffin separations performed by the molecular sieves has been investigated by numerous researchers.

Studies with various molecular sieve such as 13X, 5A and ion-exchanged molecular sieve indicated that olefins and paraffins can be separated using an equilibrium adsorption step followed by a stepwise thermal regeneration (variable-temperature stepwise desorption, VTSD). Based on this technology a commercial process for olefin/paraffin separation is licensed by UOP Inc. The pilot plant results indicate that high recoveries (99.7 wt %) and purities (99.6 wt % on a desorbent-free basis) can be obtained. However, An economic comparison indicated that the VTSD energy costs were lower, but capital costs were higher, than a comparable distillation process ((a) Shu, C.; Kulvaranon, S.; Findley, M.; Liapis, A. Separations Technology 1990, 1, 18(b) Kulvaranon, S.; Findley, M. E.; Liapis, A. I. Industrial & engineering chemistry research 1990, 29, 106(c) Schoellner, R.; Mueller, U. Adsorption science & technology 1986, 3, 167; Tajbl, D.; Kanofsky, J.; Braband, J. Energy Process./Can. 1980, 72 (5), 61 1987, 63; Faiz, R.; Li, K. Desalination 2012, 287, 82).

Besides the physical absorption methods, the chemical absorption technologies have also been investigated. $\pi$-complexation absorption is a well know technique. The basis of this chemisorption is the olefin $\pi$ bonds interact with the $\sigma$ and $\pi$ bonds of the metal. Copper and silver ions are commonly used as complexing metals. The separation factors of $\pi$-complexation absorption can achieve 17/1 for ethylene/ethane and 10/1 for propylene/propane using cuprous diketonate in an $\alpha$-methylstyrene solvent. The agent is also capable of separating branched and unbranched olefins based on steric effects (Ho, W. W.; Doyle, G.; Savage, D. W.; Pruett, R. L. Industrial & engineering chemistry research 1988, 27, 334). As a shortage, this process requires significant feed pretreatment to remove feed contaminants, such as $H_2S$ and $CO_2$, which will interfere with the complex formation or destroy the complexing agent.

On the other hand, the metal-based facilitated transport membranes have been introduced into olefin/paraffin separation process. The olefin forms a reversible complex with the metal carrier. The complex migrates across the swollen membrane film releasing the olefin on the opposite side of the membrane (Eldridge, R. B. Industrial & engineering chemistry research 1993, 32, 2208). A 60-day continuous test of silver-impregnated commercial hollow fiber membrane indicated that the permeation rate decreased by a factor of 5 during the test, with a steady decrease the first 15 days of the test (Hughes, R.; Mahoney, J.; Steigelmann, E. Recent Developments in Separation Science 1986, 9, 173). The olefin selectivity remained above 92% for the entire test. The loss in performance was attributed to loss of water and Ag+ from the membrane film. As the $\pi$-complexation absorption technology, facilitated transport membranes also have similar contamination problems, along with the need for water content control. The addition of water into the separating device's feed stream can also cause problems in downstream processes. And water must be removed from the olefin stream prior to introduction into the polymerization reactor when the process applied in the polyolefin plant. Hence, the cost of the separation system is raised by this dehydration step. To sum up, physical adsorption olefin loadings are low, and the process and regeneration cycles are complicated. Chemical adsorption systems suffer from low olefin loadings (<5 wt %) and contamination problems. With all these complications and problems, development of sustainable and economically viable olefin/paraffin separation processes is becoming increasingly important.

US 3 527 837 A discloses a process for the recovery of isobutylene comprising contacting a reactor effluent comprising isobutane, isobutylene and straight chain four carbon hydrocarbons, in a solvent contacting zone with a solvent which selectively dissolves isobutylene in preference to n-butane and isobutane. A particularly preferred solvent is furfural or a furfural-water mixture. Other solvents are acetone, acetonitrile, dimethyl formamide, dimethyl sulphoxide, N-methyl pyrrolidone, methylethyl ketone, dimethyl acetamide, acetonitrile, 3-methoxypropionitrile, and mixtures of these solvents with water.

US 5 085 741 A discloses a process for separating propylene from propane by extractive distillation of a feed consisting essentially of propylene and propane, said process employing a solvent which consists essentially of propylene carbonate.

## SUMMARY OF THE INVENTION

To address issues associated with prior separation technologies, such as energy-intensive distillation processes and under-developed physical and chemical adsorption technologies, the invention provides a process for separating gaseous hydrocarbons which is both inexpensive, effective in separating gaseous hydrocarbons and can be performed at low temperatures, consuming a relatively low amount of energy. In the process, an extractant comprising a polar solvent is used to alter the relative volatility of the hydrocarbon components to be separated, allowing a first, purified hydrocarbon component to be obtained (for example at the top of a distillation column) and a second component together with the extractant (e.g. at the bottom of the column). The second component can then easily be separated from the extractant.

Accordingly, the invention provides a process as defined in claim 1 for separating one or more hydrocarbons from a mixture of hydrocarbons.

## BRIEF DESCRIPTION OF THE FIGURES

Fig. 1 is a schematic illustration of the continuous distribution of bond strengths, showing the span from Simple Fluids, through Association Solutions to the formation of Chemical Bonds, and the continuous evolution of bond energies from ca. 0.1 through to > 1000 kJ mol$^{-1}$.

Fig. 2 is a schematic illustration of the hydrogen-bonding interaction between ammonia and water molecules.

Fig. 3 is a photograph of the gas separation equipment employed in the Example.

Fig. 4 is a table of the separation performance results for solvent samples (i) to (v) tested in the Example, for separating model catalytic cracking gas mixture (MCCGM).

Fig. 5 is a graph showing MCCGM separation performance in the adsorption stage.

Fig. 6 is a graph showing MCCGM separation performance in the desorption stage.

Fig. 7 is a table of total adsorbed gas volumes upon treating MCCGM with solvent samples (i) to (v).

Fig. 8 is a graph of the total adsorbed gas volume vs. Polarity Index (for MCCGM).

Fig. 9 is a table of the separation performance results for solvent samples (vi) to (x) tested in the Example, for separating model thermal cracking gas mixture (MTCGM).

Fig. 10 is a graph showing MTCGM separation performance in the adsorption stage.

Fig. 11 is a graph showing MTCGM separation performance in the desorption stage.

Fig. 12 is a table of total adsorbed gas volumes upon treating MTCGM with solvent samples (vi) to (x).

Fig. 13 is a graph of the total adsorbed gas volume vs. Hildebrand Parameter (MTCGM).

Fig. 14 is a table showing the fraction of other gases desorbed from samples (vi) to (x) in the desorption stage after treatment with MTCGM.

## DETAILED DESCRIPTION OF THE INVENTION

The term "hydrocarbon", as used herein, takes its normal meaning. Thus, a hydrocarbon is a compound which consists only of carbon and hydrogen. For the avoidance of doubt, hydrocarbons include straight-chained and branched, saturated and unsaturated aliphatic hydrocarbon compounds, including alkanes, alkenes, and alkynes, as well as saturated and unsaturated cyclic aliphatic hydrocarbon compounds, including cycloalkanes, cycloalkenes and cycloalkynes. Hydrocarbons also include aromatic hydrocarbons, i.e. hydrocarbons comprising one or more aromatic rings. The aromatic rings may be monocyclic or polycyclic. Aliphatic hydrocarbons which are substituted with one or more aromatic hydrocarbons, and aromatic hydrocarbons which are substituted with one or more aliphatic hydrocarbons, are also of course encompassed by the term "hydrocarbon" (such compounds consisting only of carbon and hydrogen) as are straight-chained or branched aliphatic hydrocarbons that are substituted with one or more cyclic aliphatic hydrocarbons, and cyclic aliphatic hydrocarbons that are substituted with one or more straight-chained or branched aliphatic hydrocarbons.

A "$C_{n-m}$ hydrocarbon", where n and m are integers, is a hydrocarbon, as defined above, having from n to m carbon atoms. For instance, a $C_{1-10}$ hydrocarbon is a hydrocarbon as defined above which has from 1 to 10 carbon atoms, and a $C_{1-4}$ hydrocarbon is a hydrocarbon as defined above which has from 1 to 4 carbon atoms.

The term "alkane", as used herein, refers to a linear or branched chain saturated hydrocarbon compound. A "$C_{n-m}$ alkane" refers to an alkane having from n to m carbon atoms. Thus, for instance, an alkane may be a $C_{1-20}$ alkane, i.e. an alkane having from 1 to 20 carbon atoms, or for instance a $C_{1-10}$ alkane, i.e. an alkane having from 1 to 10 carbon atoms. It may for instance be a $C_{1-8}$ alkane, a $C_{1-6}$ alkane, a $C_{1-5}$ alkane, or a $C_{1-4}$ alkane, or for instance a $C_{2-20}$ alkane, a $C_{2-10}$ alkane, a $C_{2-8}$ alkane, a $C_{2-6}$ alkane, a $C_{2-5}$ alkane, a $C_{2-4}$ alkane, or a $C_{2-3}$ alkane. It is often a $C_{1-4}$ alkane, $C_{1-3}$ alkane or $C_{2-3}$ alkane in the present invention. Examples of smaller alkanes, e.g. of a $C_{1-10}$ alkane, are for instance, methane, ethane, propane, butane, isobutane, pentane, isopentane, hexane, methylpentane, dimethylbutane, heptane, methylhexane, dimethylpentane, octane, methylheptane, dimethylhexane, trimethylpentane, nonane, decane. The term "n-alkane" as used herein, refers to a straight chain alkane. The term "i-alkane" as used herein, refers to a branched chain alkane. Alkanes such as dimethylbutane may be one or more of the possible isomers of this compound. Thus, dimethylbutane includes 2,3-dimethybutane and 2,2-dimethylbutane. This also applies for all hydrocarbon compounds referred to herein including cycloalkane, alkene, cylcoalkene.

The term "cycloalkane", as used herein, refers to a saturated cyclic aliphatic hydrocarbon compound. A cycloalkane may for instance be a $C_{3-20}$ cycloalkane, a $C_{3-10}$ cycloalkane, a $C_{3-8}$ cycloalkane, or a $C_{3-4}$ cycloalkane. Examples of a $C_{3-8}$ cycloalkane include cyclopropane, cyclobutane, cyclopentane, cyclohexane, methylcyclopentane, cycloheptane, methylcyclohexane, dimethylcyclopentane and cyclooctane. The terms "cycloalkane" and "naphthene" may be used interchangeably.

The term "alkene", as used herein, refers to a linear or branched chain hydrocarbon compound comprising one or more double bonds. A "$C_{n-m}$ alkene" refers to an alkene having from n to m carbon atoms. Thus, for instance, an alkene may be a $C_{2-20}$ alkene, i.e. an alkene having from 2 to 20 carbon atoms, or for instance a $C_{2-10}$ alkane, i.e. an alkane having from 2 to 10 carbon atoms. It may for instance be a $C_{2-8}$ alkane, a $C_{2-6}$ alkane, a $C_{2-5}$ alkane, a $C_{2-4}$ alkane, or a $C_{2-3}$ alkane. It is often a $C_{2-4}$ alkene or a $C_{2-3}$ alkene in the present invention. Examples of smaller alkenes,

e.g. of $C_{2-12}$ alkenes are ethene (i.e.ethylene), propene (i.e. propylene), butene, pentene, methylbutene, hexene, methylpentene, dimethylbutene, heptene, methylhexene, dimethylpentene, octene, methylheptene, nonene, decene, undecene and dodecene. Alkenes typically comprise one or two double bonds. The terms "alkene" and "olefin" may be used interchangeably. The one or more double bonds may be at any position in the hydrocarbon chain. The alkenes may be cis- or trans-alkenes (or as defined using E- and Z- nomenclature). An alkene comprising a terminal double bond may be referred to as an "alk-1-ene" (e.g. hex-1-ene), a "terminal alkene" (or a "terminal olefin"), or an "alpha-alkene" (or an "alpha-olefin"). The term "alkene", as used herein also often includes cycloalkenes.

The term "cylcoalkene", as used herein, refers to partially unsaturated cyclic hydrocarbon compound. A cycloalkene may for instance be a $C_{3-20}$ cycloalkene a $C_{3-10}$ cycloalkene, a $C_{3-8}$ cycloalkene or a $C_{3-4}$ cycloalkene. Examples of a $C_{3-8}$ cycloalkene include cyclopropene, cyclobutene, cyclopentene, cyclohexene, cyclohexa-1,3-diene, methylcyclopentene, cycloheptene, methylcyclohexene, dimethylcyclopentene and cyclooctene. A cycloalkene may comprise one or two double bonds.

The term "aromatic compound", "aromatic hydrocarbon" or "aromatic hydrocarbon compound", as used herein, refers to a hydrocarbon compound comprising one or more aromatic rings. The aromatic rings may be monocyclic or polycyclic. Typically, an aromatic compound comprises a benzene ring. An aromatic compound may for instance be a $C_{6-14}$ aromatic compound, a $C_{6-12}$ aromatic compound or a $C_{6-10}$ aromatic compound. Examples of $C_{6-14}$ aromatic compounds are benzene, toluene, xylene, ethylbenzene, methylethylbenzene, diethylbenzene, naphthalene, methylnaphthalene, ethylnaphthalene and anthracene. The terms "aromatic compounds", "aromatics" and "arenes" may be used interchangeably.

The term "alcohol", as used herein, refers to an organic compound which comprises one or more hydroxy (-OH) groups. Typically, an alcohol is an alkane (for instance a $C_{1-18}$ alkane, for example a $C_{1-14}$ alkane, a $C_{1-8}$ alkane, a $C_{1-6}$ alkane or a $C_{1-4}$ alkane) substituted with one or more hydroxy groups. Examples of alcohols include methanol, ethanol, isopropanol, propane-1,2-diol (propylene glycol) and sorbitol.

The term "monohydric alcohol" as used herein, takes its normal meaning in the art, i.e. an alcohol with a single -OH group. Examples of monohydric alcohols are methanol, ethanol, propanol, butanol and pentanol.

The term "dihydric alcohol", as used herein, takes its normal meaning in the art, i.e. an alcohol with two -OH groups. Examples of dihydric alcohols are ethane-1,2-diol (ethylene glycol) and propane-1,2-diol (propylene glycol).

The term "polyhydric alcohol", as used herein, takes its normal meaning in the art, i.e. an alcohol with two or more -OH groups. Examples of polyhydric alcohols are ethane-1,2-diol (ethylene glycol), propane-1,2-diol (propylene glycol) and sorbitol.

The term "alkyl", as used herein, refers to a linear or branched chain saturated hydrocarbon radical. An alkyl group may be a $C_{1-18}$ alkyl group, a $C_{1-14}$ alkyl group, a $C_{1-10}$ alkyl group, a $C_{1-6}$ alkyl group or a $C_{1-4}$ alkyl group. Examples of a $C_{1-10}$ alkyl group are methyl, ethyl, propyl, butyl, pentyl, hexyl, heptyl, octyl, nonyl or decyl. Examples of $C_{1-6}$ alkyl groups are methyl, ethyl, propyl, butyl, pentyl or hexyl. Examples of $C_{1-4}$ alkyl groups are methyl, ethyl, i-propyl, n-propyl, t-butyl, s-butyl or n-butyl. If the term "alkyl" is used without a prefix specifying the number of carbons anywhere herein, it has from 1 to 6 carbons.

The term "cycloalkyl", as used herein, refers to a saturated or partially unsaturated cyclic hydrocarbon radical. A cycloalkyl group may be a $C_{3-10}$ cycloalkyl group, a $C_{3-8}$ cycloalkyl group or a $C_{3-6}$ cycloalkyl group. Examples of a $C_{3-8}$ cycloalkyl group include cyclopropyl, cyclobutyl, cyclopentyl, cyclohexyl, cyclohexenyl, cyclohex-1,3-dienyl, cycloheptyl and cyclooctyl. Examples of a $C_{3-6}$ cycloalkyl group include cyclopropyl, cyclobutyl, cyclopentyl, and cyclohexyl.

The term "alkenyl", as used herein, refers to a linear or branched chain hydrocarbon radical comprising one or more double bonds. An alkenyl group may be a $C_{2-18}$ alkenyl group, a $C_{2-14}$ alkenyl group, a $C_{2-10}$ alkenyl group, a $C_{2-6}$ alkenyl group or a $C_{2-4}$ alkenyl group. Examples of a $C_{2-10}$ alkenyl group are ethenyl (vinyl), propenyl, butenyl, pentenyl, hexenyl, heptenyl, octenyl, nonenyl or decenyl. Examples of $C_{2-6}$ alkenyl groups are ethenyl, propenyl, butenyl, pentenyl or hexenyl. Examples of $C_{2-4}$ alkenyl groups are ethenyl, i-propenyl, n-propenyl, s-butenyl or n-butenyl. Alkenyl groups typically comprise one or two double bonds.

The term "alkynyl", as used herein, refers to a linear or branched chain hydrocarbon radical comprising one or more triple bonds. An alkynyl group may be a $C_{2-18}$ alkynyl group, a $C_{2-14}$ alkynyl group, a $C_{2-10}$ alkynyl group, a $C_{2-6}$ alkynyl group or a $C_{2-4}$ alkynyl group. Examples of a $C_{2-10}$ alkynyl group are ethynyl, propynyl, butynyl, pentynyl, hexynyl, heptynyl, octynyl, nonynyl or decynyl. Examples of $C_{1-6}$ alkynyl groups are ethynyl, propynyl, butynyl, pentynyl or hexynyl. Alkynyl groups typically comprise one or two triple bonds.

The term "aryl", as used herein, refers to a monocyclic, bicyclic or polycyclic aromatic ring which contains from 6 to 14 carbon atoms, typically from 6 to 10 carbon atoms, in the ring portion. Examples include phenyl, naphthyl, indenyl and indanyl groups. The term "aryl group", as used herein, includes heteroaryl groups. The term "heteroaryl", as used herein, refers to monocyclic or bicyclic heteroaromatic rings which typically contains from six to ten atoms in the ring portion including one or more heteroatoms. A heteroaryl group is generally a 5- or 6-membered ring, containing at least one heteroatom selected from O, S, N, P, Se and Si. It may contain, for example, one, two or three heteroatoms. Examples of heteroaryl groups include pyridyl, pyrazinyl, pyrimidinyl, pyridazinyl, furanyl, thienyl, pyrazolidinyl, pyrrolyl, oxazolyl, oxadiazolyl, isoxazolyl, thiadiazolyl, thiazolyl, isothiazolyl, imidazolyl, pyrazolyl, quinolyl and isoquinolyl.

The terms "alkylene", "cycloalkylene", "alkenylene", "alkynylene", and "arylene", as used herein, refer to bivalent groups obtained by removing a hydrogen atom from an alkyl, cycloalkyl, alkenyl, alkynyl, or aryl group, respectively. An alkylene group may be a $C_{1-18}$ alkylene group, a $C_{1-14}$ alkylene group, a $C_{1-10}$ alkylene group, a $C_{1-6}$ alkylene group or a $C_{1-4}$ alkylene group. Examples of $C_{1-6}$ alkylene groups are methylene, ethylene, propylene, butylene, pentylene and hexylene. A cycloalkylene group may be a $C_{3-10}$ cycloalkylene group, a $C_{3-8}$ cycloalkylene group or a $C_{3-6}$ cycloalkylene group. Examples of $C_{3-6}$ cycloalkylene groups include cyclopentylene and cyclohexylene. An alkenylene group may be a $C_{2-18}$ alkenylene group, a $C_{2-14}$ alkenylene group, a $C_{2-10}$ alkenylene group, a $C_{2-6}$ alkenylene group or a $C_{2-4}$ alkenylene group. Examples of a $C_{2-4}$ alkenylene group include ethenylene (vinylene), propenylene and butenylene. An alkynylene group may be a $C_{2-18}$ alkynylene group, a $C_{2-14}$ alkynylene group, a $C_{2-10}$ alkynylene group, a $C_{2-6}$ alkynylene group or a $C_{2-4}$ alkynylene group. Examples of a $C_{2-4}$ alkynylene group include ethynylene and propynylene. Examples of arylene groups include phenylene and a diradical derived from thiophene. For alkylene, cycloalkylene, alkenylene, alkynylene, and arylene, these groups may be bonded to other groups at any two positions on the group. Thus, propylene includes - $CH_2CH_2CH_2$- and -$CH_2CH(CH_3)$-, and phenylene includes ortho-, meta- and para-phenylene.

The term "substituted", as used herein in the context of substituted organic compounds, refers to an organic compound which bears one or more substituents selected from $C_{1-10}$ alkyl, aryl (as defined herein), cyano, amino, nitro, $C_{1-10}$ alkylamino, di($C_{1-10}$)alkylamino, arylamino, diarylamino, aryl($C_{1-10}$)alkylamino, amido, acylamido, hydroxy, oxo, halo, carboxy, ester, acyl, acyloxy, $C_{1-10}$ alkoxy, aryloxy, halo($C_{1-10}$)alkyl, sulfonic acid, thiol, $C_{1-10}$ alkylthio, arylthio, sulfonyl, phosphoric acid, phosphate ester, phosphonic acid and phosphonate ester. Typically, the one or more substituents are selected from cyano, amino, nitro, amido, acylamido, hydroxy, oxo, halo, carboxy, ester, acyl, acyloxy, sulfonic acid, thiol, sulfonyl, phosphoric acid, phosphate ester, phosphonic acid and phosphonate ester When a compound is substituted, it may bear 1, 2, 3 or 4 substituents. For instance, a substituted compound may have 1, 2 or 3 substitutents, or for example 1 or 2 substitutents.

The process of the invention as defined in claim 1 is a process for separating one or more hydrocarbons from a gaseous mixture of hydrocarbons, which gaseous mixture comprises: (a) one or more first hydrocarbons, and (b) one or more further hydrocarbons which are other than the one or more first hydrocarbons.

The term "gaseous mixture of hydrocarbons", as used herein, refers to a composition which comprises a mixture of two or more hydrocarbons which are in the gas state at standard ambient temperature and pressure (SATP). Standard ambient temperature and pressure (SATP) means a temperature of 298.15 K (25 °C) and at 100,000 Pa (1 bar, 14.5 psi, 0.9869 atm).

The two or more hydrocarbons in the mixture are not necessarily therefore in the gas state under the conditions (i.e. the temperature and pressure) under which the process of the invention is carried out, because the process may for example be carried out at an elevated pressure (i.e. at a pressure above that of SATP) and/or at a reduced temperature (i.e. at a pressure below that of SATP) such that one or more of the two or more hydrocarbons in the mixture may not be in the gaseous state under those conditions, but may, for instance, be in the liquid state instead. In some embodiments of the process of the invention, however, that is not the case and the the two or more hydrocarbons in the mixture are in the gas state under the conditions (i.e. at the temperature and pressure) under which the process of the invention is carried out. The process may for instance be carried out at or near standard ambient temperature and at pressures which are above standard ambient pressure but at which the two or more hydrocarbons are still in the gaseous state. The Example section herein, for instance, describes experiments in which the process is carried out at ambient temperature and at a gauge pressure pressure of 2 bar (i.e. 2 bar above atmospheric pressure).

The one or more first hydrocarbons comprise one or more alkenes. The one or more first hydrocarbons may for instance comprise one or more $C_{2-4}$ alkenes.

The one or more further hydrocarbons comprise one or more alkanes. The one or more further hydrocarbons may for instance comprise one or more $C_{1-4}$ alkanes.

In the process of the invention, alkenes may be selectively extracted from a mixture of alkenes and alkanes using the extractant, meaning that the process may be used for separating olefins from paraffins. This is useful, for instance, in separating olefins from paraffins in gaseous mixtures obtained by petroleum cracking, for instance in separating olefins from paraffins in gaseous mixtures obtained by thermal and/or catalytic cracking of petroleum.

Thus, the one or more first hydrocarbons comprise one or more alkenes, and the one or more further hydrocarbons comprise one or more alkanes. The alkanes may for instance be $C_{1-4}$ alkanes and the alkenes may be $C_{2-4}$ alkenes. Often, for instance, the alkanes are $C_{1-3}$ alkanes and the alkenes are $C_{2-3}$ alkenes. The alkanes may for instance be $C_{2-3}$ alkanes and the alkenes may be $C_{2-3}$ alkenes.

Thus, the one or more first hydrocarbons may comprise one or more $C_{2-4}$ alkenes, and the one or more further hydrocarbons may comprise one or more $C_{1-4}$ alkanes. Often, for instance, the one or more first hydrocarbons comprise one or more $C_{2-3}$ alkenes, and the one or more further hydrocarbons may comprise one or more $C_{1-3}$ alkanes. The one or more first hydrocarbons may for example comprise one or more $C_{2-3}$ alkenes, and the one or more further hydrocarbons may comprise one or more $C_{2-3}$ alkanes. For instance, the one or more first hydrocarbons may comprise ethylene and propylene and the one or more further hydrocarbons may comprise ethane and propane. The one or more first hydrocarbons may for example comprise ethylene and propylene, and the one or more further hydrocarbons may comprise methane, ethane and propane.

The one or more first hydrocarbons may for instance be selected from alkenes and cycloalkenes, and the one or more further hydrocarbons may be selected from alkanes and cycloalkanes. The alkanes and cycloalkanes may for instance be $C_{1-4}$ alkanes and $C_{3-4}$ cycloalkanes respectively, and the alkenes and cycloalkenes may be $C_{2-4}$ alkenes and $C_{3-4}$ cycloalkenes respectively.

Typically, the one or more first hydrocarbons are selected from alkenes, and the one or more further hydrocarbons are selected from alkanes. The alkanes may for instance be $C_{1-4}$ alkanes and the alkenes may be $C_{2-4}$ alkenes.

Often, for instance, the alkanes are $C_{1-3}$ alkanes and the alkenes are $C_{2-3}$ alkenes. For instance, the one or more first hydrocarbons may be ethylene and propylene and the one or more further hydrocarbons may be methane, ethane and propane.

The alkanes may for instance be $C_{2-3}$ alkanes and the alkenes may be $C_{2-3}$ alkenes. Thus, the one or more first hydrocarbons may be ethylene and propylene and the one or more further hydrocarbons may be ethane and propane.

Often, the gaseous mixture of hydrocarbons is a gaseous mixture which is obtainable by catalytic cracking of petroleum. The gaseous mixture of hydrocarbons often therefore comprises ethylene, ethane, propylene and propane.

The gaseous mixture of hydrocarbons may for instance comprise methane, ethylene, ethane, propylene and propane.

As discussed above, the gaseous mixture of hydrocarbons is a composition which comprises a mixture of two or more hydrocarbons which are in the gas state at SATP. However, that composition may comprise other components, in addition to the two or more hydrocarbons. It may for instance further comprise an inert gas, hydrogen gas, carbon monoxide and/or one or more contaminants. For example, the gaseous mixture of hydrocarbons may further comprise an inert gas. The inert gas may comprise nitrogen or a noble gas, such as argon. Additionally or alternatively, the gaseous mixture of hydrocarbons may further comprise hydrogen gas. Additionally or alternatively, the gaseous mixture of hydrocarbons may further comprise carbon monoxide. Additionally or alternatively, the gaseous mixture of hydrocarbons may further comprise carbon dioxide. Additionally or alternatively, the gaseous mixture of hydrocarbons may further comprise a contaminant, such as for instance hydrogen sulfide and/or one or more organosulfur compounds.

The gaseous mixture of hydrocarbons often therefore comprises ethylene, ethane, propylene, propane and an inert gas. The inert gas typically comprises nitrogen or a noble gas, for instance argon. More typically, it comprises nitrogen.

The gaseous mixture of hydrocarbons may for instance comprise methane, ethylene, ethane, propylene and propane, and an inert gas. The inert gas typically comprises nitrogen or a noble gas, for instance argon. More typically, the inert gas comprises nitrogen.

Often, the gaseous mixture of hydrocarbons is a gaseous mixture which is obtainable by thermal cracking of petroleum. The gaseous mixture of hydrocarbons often therefore comprises hydrogen, carbon monoxide, methane, ethylene, ethane, propylene and propane.

The gaseous mixture of hydrocarbons may further comprise an inert gas. The inert gas typically comprises nitrogen or a noble gas, for instance argon. More typically, the inert gas comprises nitrogen. The gaseous mixture of hydrocarbons may therefore comprise hydrogen, carbon monoxide, methane, ethylene, ethane, propylene, propane and an inert gas. Usually, the inert gas comprises nitrogen.

Higher molecular weight alkenes may be selectively extracted using the extractant from a mixture of those alkanes with other alkanes of a lower molecular weight, meaning that the process may be used for separating paraffins of differing molecular weight. This is useful for instance in the separation of the paraffins in natural gas, coal bed gas and shale gas.

The invention can also of course be employed for separating olefins from paraffins and for separating paraffins of differing molecular weight, all at the same time.

Thus, in the process of the invention, the one or more first hydrocarbons may comprise one or more first alkanes or cycloalkanes and one or more alkenes or cycloalkenes, and the one or more further hydrocarbons may comprise one or more further alkanes or cycloalkanes, wherein each of the one or more first alkanes or cycloalkanes has a higher molecular weight than each of the one or more further alkanes or cycloalkanes.

The first and further alkanes and cycloalkanes may for instance be $C_{1-4}$ alkanes and $C_{3-4}$ cycloalkanes respectively, provided of course that each of the one or more first alkanes or cycloalkanes has a higher molecular weight than each of the one or more further alkanes or cycloalkanes. The alkenes and cycloalkenes may be $C_{2-4}$ alkenes and $C_{3-4}$ cycloalkenes respectively.

Typically, the one or more first hydrocarbons comprise one or more first alkanes and one or more alkenes, and the one or more further hydrocarbons comprise one or more further alkanes, wherein each of the one or more first alkanes has a higher molecular weight than each of the one or more further alkanes.

The one or more first alkanes and one or more further alkanes may be $C_{1-4}$ alkanes, provided of course that each of the one or more first alkanes has a higher molecular weight than each of the one or more further alkanes. The one or more alkenes may be $C_{2-4}$ alkenes.

Thus, the one or more first hydrocarbons may for instance comprise one or more $C_{2-4}$ alkenes and one or more $C_{2-4}$ alkanes, and the one or more further hydrocarbons may comprise methane.

In process of the invention, the gaseous mixture of hydrocarbons may comprise natural gas, shale gas, coal bed gas, biogas, refinery gas, or a gas which is obtainable by catalytic and/or thermal cracking of petroleum.

The process of the invention as defined hereinbefore comprises contacting the gaseous mixture of hydrocarbons with said extractant suitable for extracting the one or more first hydrocarbons, and thereby forming: (i) a first phase comprising the extractant and the one or more first hydrocarbons; and (ii) a second phase comprising said gaseous mixture having a reduced content of the one or more first hydrocarbons.

The extractant employed in the process of the invention is typically also suitable for extracting sulfur-containing impurities from hydrocarbon mixtures, such as organosulfur compounds and hydrogen sulfide.

Thus, in the process of the invention, including in any embodiment thereof defined herein, the gaseous mixture of hydrocarbons may further comprise hydrogen sulfide and/or one or more organosulfur compounds, the first phase may further comprise said hydrogen sulfide and/or said one or more organosulfur compounds, and the second phase may have a reduced content of said hydrogen sulfide and/or said one or more organosulfur compounds.

Often, in the process of the invention, including any embodiment thereof defined herein, the gaseous mixture further comprises one or more organosulfur compounds, the first phase further comprises the one or more organosulfur compounds, and the second phase has a reduced content of the one or more organosulfur compounds.

The term "reduced content", as used herein in relation to a particular component of the gaseous mixture of hydrocarbons, refers to a reduction of the content of that component relative to the untreated gaseous mixture of hydrocarbons.

The extractant employed in the process of the invention is suitable for extracting the one or more first hydorcarbons. It is a solvent system which selectively extracts the one or more first hydrocarbons. The solvent system may comprise a single solvent or a plurality of solvents. The extractant is also referred to herein as the "extraction solvent". The terms "extractant" and "extraction solvent", as used herein, shall be taken to have an identical meaning and to be interchangeable.

The extractant comprises a polar solvent, in particular a polar organic solvent which is the alcohol methanol. The extraction solvent may comprise two or more polar organic solvents. The extraction solvent may consist essentially of one or more polar organic solvents. The extraction may consist essentially of a single polar organic solvent. However, the extraction solvent may also be a binary solvent or a multinary solvent as discussed below.

A carbonate which the extraction solvent may comprise may be any $C_{3-10}$ carbonate. A carbonate typically has the structure alkyl-OC(O)O-alkyl. Examples of the carbonate that the extraction solvent may comprise include dimethylcarbonate, ethylmethylcarbonate and diethyl carbonate. The carbonate may be propylene carbonate or trimethylene carbonate.

The extractant employed in the present invention may for instance comprise a polar protic organic solvent, both a polar protic organic solvent and a carbonate, both a polar protic organic solvent and a sulfoxide, or a first polar protic organic solvent and a second polar protic organic solvent which is different from the first (i.e. two different polar protic organic solvents). For instance, the extractant may comprise: a polar protic organic solvent, or both a polar protic organic solvent and a carbonate. The carbonate and sulfoxide in these embodiments may be as further defined anywhere herein, and the polar protic organic solvent is methanol.

The extraction solvent comprises the alcohol methanol. The extraction solvent comprises said alcohol in an amount of greater than of equal to 40 wt%. Often, the extraction solvent comprises one or more alcohols in an amount of greater than or equal to 80 wt%. The extraction solvent may comprise methanol in an amount of greater than or equal to 50 wt%.

The extractant may comprise: an alcohol, or an alcohol and a carbonate. The carbonate may be as further defined anywhere herein. The alcohol is methanol. Usually, the carbonate is dimethylcarbonate (DMC) or propylene carbonate.

Often, the extractant employed in the present invention comprises DMC and a second solvent which is methanol.

Preferably, the extractant comprises: (i) methanol; or (ii) methanol and DMC.

The Scatchard-Hildebrand theory has two basic underlying assumptions: (i) the forces between molecules are dispersion forces (often called van der Waals' forces); and (ii) the entropy change on forming the solution has the same value as for an ideal solution. Based on this theory Hildebrand proposed a definition for a "solubility parameter" that would provide a systemic description of the miscibility behavior of solvents (A F Barton Chemical Reviews 1975 75 No 6 p 731). This solubility parameter is defined as the square root of the cohesive energy density, the heat of vaporization divided by the molar volume.

The other solubility parameter is the Hansen solubility parameters which are proposed by Hansen. The basis of these Hansen solubility parameters is that the total energy of vaporization of a liquid consists of several individual parts. These arise from (atomic) dispersion forces, (molecular) permanent dipole-permanent dipole forces, and (molecular) hydrogen bonding (electron exchange). That means the Hildebrand solubility parameter has been divided into these three dimensions by the Hansen solubility parameters.

The three dimensions from the Hansen solubility parameters are derived from atomic force and have also been called dispersion interactions $\delta_d$ in the literature. The permanent dipole-permanent dipole interactions cause a second type of cohesion energy, the polar cohesive energy $\delta_p$. These are inherently molecular interactions and are found in most molecules to one extent or another. The dipole moment is primary parameter used to calculate these interactions. The third major cohesive energy source is hydrogen bonding, which can be called more generally an electron exchange parameter $\delta_h$. Hydrogen bonding is a molecular interaction and resembles the polar interactions in this respect. The basis of this type of cohesive energy is attraction among molecules because of the hydrogen bonds.

The relation between the Hildebrand and Hansen solubility parameters can be described by the following equation.

$$\delta^2 = (\delta_d)^2 + (\delta_p)^2 + (\delta_h)^2$$

Here, $\delta$ is the Hildebrand solubility parameter, $\delta_d$ is the nonpolar interactions (dispersion interactions) of the Hansen solubility parameter, $\delta_p$ is the dipole-permanent dipole interactions (the polar cohesive energy) of the Hansen solubility parameter, $\delta_h$ is the hydrogen bonding interactions (electron exchange parameter) of the Hansen solubility parameter.

A comprehensive collection of Hildebrand and Hansen solubility parameters have been given by Barton (see Barton, A. F., CRC handbook of solubility parameters and other cohesion parameters, CRC press, 1991 and Reichardt, C.; Welton, T., Solvents and solvent effects in organic chemistry, John Wiley & Sons: 2011).

A polar solvent may have a Hildebrand solubility parameter ($\delta$) which is equal to or greater than 12 MPa$^{1/2}$, or for instance which is equal to or greater than 14 MPa$^{1/2}$.

A polar solvent may have a Hildebrand solubility parameter ($\delta$) which is equal to or greater than 18 MPa$^{1/2}$.

A polar solvent employed may for instance have a Hildebrand solubility parameter ($\delta$) which is less than or equal to 40 MPa$^{1/2}$.

Thus, a polar solvent may have a Hildebrand solubility parameter ($\delta$) of from 18 MPa$^{1/2}$ to 40 MPa$^{1/2}$, for instance from 20 MPa$^{1/2}$ to 30 MPa$^{1/2}$.

A polar solvent may have a Hansen non-polar interaction solubility parameter of from 12.0 to 25.0 MPa$^{1/2}$, for instance from 15.5 to 22.0 MPa$^{1/2}$; a Hansen polar cohesive energy solubility parameter $\delta_p$ of from 0.0 to 20.0 MPa$^{1/2}$, for instance from 5.0 to 19.0 MPa$^{1/2}$; and a Hansen electron exchange parameter solubility parameter $\delta_h$ of from 0.0 to 35.0 MPa$^{1/2}$, for instance from 3.0 to 25.0 MPa$^{1/2}$. Typically, the extractant has a Hildebrand solubility parameter ($\delta$) which is equal to or greater than 12 MPa$^{1/2}$, or for instance which is equal to or greater than 14 MPa$^{1/2}$.

Often, the extractant has a Hildebrand solubility parameter ($\delta$) which is equal to or greater than 18 MPa$^{1/2}$.

Usually, the extractant has a Hildebrand solubility parameter ($\delta$) which is less than or equal to 40 MPa$^{1/2}$.

Thus, the extractant typically has a Hildebrand solubility parameter ($\delta$) of from 18 MPa$^{1/2}$ to 40 MPa$^{1/2}$, and preferably from 20 MPa$^{1/2}$ to 30 MPa$^{1/2}$.

The extractant may have a Hansen non-polar interaction solubility parameter $\delta_d$ of from 12.0 to 25.0 MPa$^{1/2}$, for instance from 15.5 to 22.0 MPa$^{1/2}$; a Hansen polar cohesive energy solubility parameter $\delta_p$ of from 0.0 to 20.0 MPa$^{1/2}$, for instance from 5.0 to 19.0 MPa$^{1/2}$; and a Hansen electron exchange parameter solubility parameter $\delta_h$ of from 0.0 to 35.0 MPa$^{1/2}$, for instance from 3.0 to 25.0 MPa$^{1/2}$.

The extractant comprises a polar solvent, which is typically a polar organic solvent. The polar solvent and the extractant may be defined in terms of Snyder's polarity index, P' (L R Snyder J of Chromatography 92 (1974) 223). The polarity index, P', is well known in the art for a wide variety of solvents and mixtures of solvents, and can readily be dermined by the skilled person for a given solvent system.

A polar solvent may have a polarity index (P') which is equal to or greater than 2.

A polar solvent may for instance have a polarity index (P') which is equal to or greater than 3.

A polar solvent may for instance have a polarity index (P') which is equal to or greater than 4, preferably equal to or greater than 5, and more preferably equal to or greater than 6.

A polar solvent may have a polarity index (P') of from 2 to 10, for instance from 3 to 8.

Thus, a polar solvent may have a Hildebrand solubility parameter ($\delta$) which is equal to or greater than 12 MPa$^{1/2}$, for instance equal to or greater than 14 MPa$^{1/2}$, and a polarity index (P') which is equal to or greater than 3.

A polar solvent may for example have a Hildebrand solubility parameter ($\delta$) which is equal to or greater than 18 MPa$^{1/2}$, and a polarity index (P') which is equal to or greater than 3.

For instance, the polar solvent may have a Hildebrand solubility parameter ($\delta$) which is equal to or greater than 18 MPa$^{1/2}$, and a polarity index (P') which is equal to or greater than 4, for instance equal to or greater than 5, or equal to or greater than 6.

For instance, the polar solvent may have a Hildebrand solubility parameter ($\delta$) which is equal to or greater than 18 MPa$^{1/2}$, and a polarity index (P') which is from 2 to 10, for instance from 3 to 8.

A polar solvent may have a Hildebrand solubility parameter ($\delta$) which is less than or equal to 40 MPa$^{1/2}$.

Thus, a polar solvent may have a Hildebrand solubility parameter ($\delta$) of from 12 MPa$^{1/2}$ to 40 MPa$^{1/2}$, for instance from 18 MPa$^{1/2}$ to 30 MPa$^{1/2}$, and a polarity index (P') which is equal to or greater than 3.

A polar solvent may for instance have a Hildebrand solubility parameter ($\delta$) of from 12 MPa$^{1/2}$ to 40 MPa$^{1/2}$, for instance from 18 MPa$^{1/2}$ to 30 MPa$^{1/2}$, and a polarity index (P') which is equal to or greater than 4.

A polar solvent may for instance have a Hildebrand solubility parameter ($\delta$) of from 12 MPa$^{1/2}$ to 40 MPa$^{1/2}$, for instance from 18 MPa$^{1/2}$ to 30 MPa$^{1/2}$, and a polarity index (P') which is equal to or greater than 5.

A polar solvent may for instance have a Hildebrand solubility parameter ($\delta$) of from 12 MPa$^{1/2}$ to 40 MPa$^{1/2}$, or from 18 MPa$^{1/2}$ to 30 MPa$^{1/2}$, and a polarity index (P') which is equal to or greater than 6.

A polar solvent may for instance have a Hildebrand solubility parameter ($\delta$) of from 12 MPa$^{1/2}$ to 40 MPa$^{1/2}$, for instance from 18 MPa$^{1/2}$ to 30 MPa$^{1/2}$, and a polarity index (P') which is from 2 to 10.

A polar solvent may for instance have a Hildebrand solubility parameter ($\delta$) of from 12 MPa$^{1/2}$ to 40 MPa$^{1/2}$, for instance from 18 MPa$^{1/2}$ to 30 MPa$^{1/2}$, and a polarity index (P') which is from 3 to 8.

Typically, in the process of the invention, the extractant has a polarity index (P') which is equal to or greater than 2.

More typically, the extractant has a polarity index (P') which is equal to or greater than 3.

The extractant may for instance have a polarity index (P') which is equal to or greater than 4, preferably equal to or greater than 5, and more preferably equal to or greater than 6.

The extractant preferably has a polarity index (P') of from 2 to 10, more preferably from 3 to 8.

Thus, the extractant may have a Hildebrand solubility parameter ($\delta$) which is equal to or greater than 12 MPa$^{1/2}$, for instance equal to or greater than 14 MPa$^{1/2}$, and a polarity index (P') which is equal to or greater than 3.

The extractant may for example have a Hildebrand solubility parameter ($\delta$) which is equal to or greater than 18 MPa$^{1/2}$, and a polarity index (P') which is equal to or greater than 3.

Preferably, for instance, the extractant has a Hildebrand solubility parameter ($\delta$) which is equal to or greater than 18 MPa$^{1/2}$, and a polarity index (P') which is equal to or greater than 4, preferably equal to or greater than 5, and more preferably equal to or greater than 6.

Preferably, for instance, the extractant has a Hildebrand solubility parameter ($\delta$) which is equal to or greater than 18 MPa$^{1/2}$, and a polarity index (P') which is from 2 to 10, more preferably from 3 to 8.

Usually, in these embodiments, the extractant employed has a Hildebrand solubility parameter ($\delta$) which is less than or equal to 40 MPa$^{1/2}$.

Thus, the extractant employed typically has a Hildebrand solubility parameter ($\delta$) of from 12 MPa$^{1/2}$ to 40 MPa$^{1/2}$, and preferably from 18 MPa$^{1/2}$ to 30 MPa$^{1/2}$, and a polarity index (P') which is equal to or greater than 3.

The extractant employed may for instance have a Hildebrand solubility parameter ($\delta$) of from 12 MPa$^{1/2}$ to 40 MPa$^{1/2}$, and preferably from 18 MPa$^{1/2}$ to 30 MPa$^{1/2}$, and a polarity index (P') which is equal to or greater than 4.

The extractant employed may for instance have a Hildebrand solubility parameter ($\delta$) of from 12 MPa$^{1/2}$ to 40 MPa$^{1/2}$, and preferably from 18 MPa$^{1/2}$ to 30 MPa$^{1/2}$, and a polarity index (P') which is equal to or greater than 5.

The extractant employed may for instance have a Hildebrand solubility parameter ($\delta$) of from 12 MPa$^{1/2}$ to 40 MPa$^{1/2}$, and preferably from 18 MPa$^{1/2}$ to 30 MPa$^{1/2}$, and a polarity index (P') which is equal to or greater than 6.

The extractant employed may for instance have a Hildebrand solubility parameter ($\delta$) of from 12 MPa$^{1/2}$ to 40 MPa$^{1/2}$, and preferably from 18 MPa$^{1/2}$ to 30 MPa$^{1/2}$, and a polarity index (P') which is from 2 to 10.

The extractant employed may for instance have a Hildebrand solubility parameter ($\delta$) of from 12 MPa$^{1/2}$ to 40 MPa$^{1/2}$, and preferably from 18 MPa$^{1/2}$ to 30 MPa$^{1/2}$, and a polarity index (P') which is from 3 to 8.

In the process of the invention, the extractant may comprise the polar solvent methanol, and a second solvent which is different from said polar solvent and is miscible with the polar solvent. The second extractant solvent is a second polar solvent. Typically, the second extractant solvent is a second polar organic solvent. The second extractant solvent may be as further defined hereinbefore for the polar solvent, provided of course that it is a different solvent from the polar solvent.

Typically, the first and second extractant solvents are different polar organic solvents.

Typically, the second extractant solvent has a Hildebrand solubility parameter ($\delta$) as defined above for the polar solvent.

Typically, the second extractant solvent has a polarity index (P') as defined above for the polar solvent.

The extractant may for instance be a mixture of two, three, or more than three, different polar organic solvents including said methanol which are miscible with one another. Typically, all of these polar organic solvents have a Hildebrand solubility parameter ($\delta$) as defined above for the polar solvent. Usually, all of these polar organic solvents have a polarity index (P') as defined above for the polar solvent.

The extractant employed in the present invention may comprise said alcohol methanol and a carbonate, said alcohol methanol and a sulfoxide, or a first alcohol which is said methanol and a second alcohol which is different from the first alcohol (i.e. two different alcohols), wherein each solvent has a Hildebrand solubility parameter ($\delta$) as defined above and a polarity index (P') as defined above. For instance, the extractant may comprise: an alcohol which is said methanol, or said alcohol methanol and a carbonate, wherein each solvent has a Hildebrand solubility parameter ($\delta$) as defined above and a polarity index (P') as defined above. The carbonate and sulfoxide in these embodiments may be as further defined anywhere herein provided that each of these solvents has a Hildebrand solubility parameter ($\delta$) as defined above and a polarity index (P') as defined above. The alcohol is methanol (which has a Hildebrand solubility parameter ($\delta$) of 29.7 MPa$^{1/2}$ and a polarity index of 5.1). Usually, the carbonate is dimethyl carbonate (which has a Hildebrand solubility parameter ($\delta$) of 20.6 MPa$^{1/2}$ and a polarity index of 3.9) or propylene carbonate (which has a Hildebrand solubility parameter ($\delta$) of 27.2 MPa$^{1/2}$ and a polarity index of 6.1). Typically, the sulfoxide is dimethylsulfoxide (which has a Hildebrand solubility parameter ($\delta$) of 24.5 MPa$^{1/2}$ and a polarity index of 7.2).

The extractant employed in the present invention comprises methanol.

Preferably, the extractant comprises: (i) methanol; or (ii) methanol and DMC.

The process of the invention may conveniently be performed at ambient temperature.

Thus, in terms of process conditions, the step of contacting the gaseous mixture of hydrocarbons with the extractant is typically performed at a temperature (which may be referred to as a "first temperature") of from -30 °C to 60 °C.

Accordingly, often, the process of the invention comprises contacting the gaseous mixture of hydrocarbons with the extractant at a temperature (which may be referred to as a "first temperature") of from -30 °C to 60 °C.

More typically, the first temperature is from -5 °C to 45 °C, for instance from 0 °C to 40 °C, preferably from 5 °C to 35 °C. Contacting the gaseous mixture of hydrocarbons with the extractant is usually performed at room temperature, for instance, from 10 °C to 30 °C. It may for instance be performed at about standard ambient temperature, i.e. at about 25 °C.

Usually, the contacting step is performed at an elevated pressure, i.e. at an absolute pressure of greater than standard ambient pressure, i.e. greater than 1 bar.

Thus, typically, the process of the invention comprises contacting the gaseous mixture of hydrocarbons with the extractant at a pressure (which may be referred to as a "first pressure") of greater than 1 bar. The pressure referred to here is the absolute pressure rather than the guage pressure.

More typically, the first pressure is greater than 1 bar and less than or equal to 300 bar, for instance from 1.5 bar to 200 bar, preferably from 1.5 bar to 100 bar, or for instance from 2 bar to 50 bar. It is often, for instance, from 2 bar to 10 bar, for instance from 3 bar to 5 bar.

The step of contacting the gaseous mixture of hydrocarbons with the extractant is typically therefore performed at a temperature (the "first temperature") of from -30 °C to 60 °C and at a pressure ("first pressure") of greater than 1 bar and less than or equal to 300 bar.

More typically, the step of contacting the gaseous mixture of hydrocarbons with the extractant is performed at a temperature (the "first temperature") of from -5 °C to 45 °C and at a pressure ("first pressure") of from 1.5 bar to 200 bar.

The step of contacting the gaseous mixture of hydrocarbons with the extractant may for instance be performed at a temperature (the "first temperature") of from 0 °C to 40 °C and at a pressure ("first pressure") of from 1.5 bar to 100 bar.

Often, for instance, the step of contacting the gaseous mixture of hydrocarbons with the extractant is performed at a temperature (the "first temperature") of from 5 °C to 35 °C and at a pressure ("first pressure") of from 2 bar to 50 bar.

The step of contacting the gaseous mixture of hydrocarbons with the extractant may for instance be performed at a temperature (the "first temperature") of from 0 °C to 40 °C and at a pressure ("first pressure") of from 2 bar to 10 bar.

Usually, for instance, the step of contacting the gaseous mixture of hydrocarbons with the extractant is performed at a temperature (the "first temperature") of from 10 °C to 30 °C and at a pressure ("first pressure") of from 3 bar to 10 bar.

The step of contacting the gaseous mixture of hydrocarbons with the extractant causes extraction of the one or more first hydrocarbons from the gaseous mixture, generally by adsorption of the one or more first hydrocarbons to the extractant, and thereby forming:

> (i) the first phase comprising the extractant and the one or more first hydrocarbons; and
> (ii) the second phase comprising said gaseous mixture having a reduced content of the one or more first hydrocarbons.
> The first phase is typically a liquid phase, and the second phase is typically a gas phase.

The process of the invention may further comprise recovering the gaseous mixture having a reduced content of the one or more first hydrocarbons. This generally comprises separating the second phase from the first phase. The second phase comprises the gaseous mixture having a reduced content of the one or more first hydrocarbons and is typically therefore in the gaseous state, whereas the first phase comprises said extractant comprising said polar solvent and is typically therefore in the liquid state. Accordingly, the step of recovering the gaseous mixture having a reduced content of the one or more first hydrocarbons is typically a gas-liquid separation step, i.e. a simple case of separating the gas phase from the liquid phase.

The process of the invention typically further comprises recovering the one or more first hydrocarbons from the first phase. Recovering the one or more first hydrocarbons from the first phase typically comprises desorbing the one or more first hydrocarbons from the extractant. The one or more first hydrocarbons may be desorbed from the extractant by reducing the pressure which the first phase is under and/or by heating the first phase.

Accordingly, recovering the one or more first hydrocarbons from the first phase usually comprises reducing the pressure which the first phase is under and/or heating the first phase. Usually, said reducing the pressure comprises reducing the pressure which said phase is under from a first pressure to a second pressure, wherein the first pressure is as defined hereinbefore and the second pressure is less than the first pressure; and said heating comprises heating said phase to increase the temperature of the phase from a first temperature to a second temperature, wherein the first temperature is as defined hereinbefore and the second temperature is greater than the first temperature.

The pressure may be reduced in a stepwise manner, e.g. it may be reduced by 1 bar at a time, and after each pressure reduction collecting the one or more first hydrocarbons that are desorbed. Often, the one or more first hydrocarbons are recovered from the first phase firstly by reducing the pressure which the first phase is under and collecting the gas that desorbs from the first phase at the reduced pressure, e.g. in a stepwise manner, and then, once the pressure has been reduced to ambient, subsequently heating the first phase to effect desorption of any of the first hydrocarbon(s) that remain adsorbed to the extractant.

Once the one or more first hydrocarbons have been recovered from the first phase, the extractant may be re-used, to extract more of the one or more first hydrocarbons from the second phase.

Accordingly, after recovering the one or more first hydrocarbons from the first phase, the process may further comprise contacting the second phase with the extractant from the first phase, and thereby forming: (i) a further first phase comprising the extractant and said one or more first hydrocarbons; and (ii) a further second phase comprising said gaseous mixture having a further reduced content of the one or more first hydrocarbons.

In terms of process conditions, said contacting may be as further defined hereinbefore for the initial step of contacting the gaseous mixture of hydrocarbons with the extractant.

The process may then further comprise: (a) recovering the one or more first hydrocarbons from the further first phase.

Once the one or more first hydrocarbons have been recovered from the further first phase, the extractant may be re-used again, any number of times, to extract more of the one or more first hydrocarbons.

Thus, after recovering the one or more first hydrocarbons from the further first phase, the process may further comprise:

(b) contacting the further second phase with the extractant from the further first phase, and thereby forming: (i) a further first phase comprising the extractant and said one or more first hydrocarbons, and (ii) a further second phase comprising said gaseous mixture having a further reduced content of the one or more first hydrocarbons; and

(c) optionally repeating steps (a) and (b) one or more times.

The term "further reduced content", as used herein, refers to a reduction of the content of the one or more first hydrocarbons relative to the second phase (or the further second phase, as the case may be) prior to treatment with the extractant.

In terms of process conditions, said contacting in step (b) may be as further defined hereinbefore for the initial step of contacting the gaseous mixture of hydrocarbons with the extractant. In particular, the temperature and pressure may be as defined hereinbefore for the initial step of contacting the gaseous mixture of hydrocarbons with the extractant.

The step of recovering the one or more first hydrocarbons from the or each further first phase typically comprises reducing the pressure which the further first phase is under and/or heating the further first phase. Usually, said reducing the pressure comprises reducing the pressure which said phase is under from a first pressure to a second pressure, wherein the first pressure is as defined hereinbefore and the second pressure is less than the first pressure; and said heating comprises heating said phase to increase the temperature of the phase from a first temperature to a second temperature, wherein the first temperature is as defined hereinbefore and the second temperature is greater than the first temperature.

The present invention is further illustrated in the Example which follows.

## EXAMPLE

To overcome the disadvantages in current extractive distillation light olefin/paraffin separation processes for the petroleum pyrolysis gas, and to develop a lower cost, less energy-intensive approach to the problem, a new solvent system- based "Extractive Distillation" approach is advanced here.

**Background**

To understand the underlying solvent extraction or solubilisation processes, one needs to consider the classified nine types of solution; these are listed in Table 1. This study is concerned with the "Gas in liquid" solution. Ammonia in Water is perhaps the most typical "Gas in liquid" solution; as the result of the intermolecular interaction between the Ammonia and Water molecules, (Yeo, G. A.; Ford, T. A. Canadian journal of chemistry 1991, 69, 632) Ammonia is very soluble in water.

**Table 1.** The nine types of solutions (From A G Sharpe, "Solubility Explained ", Education in Chemistry, 1, 75, 1964)

| Solution | Gas in gas | Liquid in gas | Solid in gas | Gas in liquid | Liquid in liquid | Solid in liquid | Gas in Liquid | Liquid in solid | Solid in solid |
|---|---|---|---|---|---|---|---|---|---|
| Example | Air | $H_2O$ (vapour)in $N_2$ | $I_2$ (vapour) in $N_2$ | $CO_2$ in $H_2O$ | Ethanol in $H_2O$ | NaCl in $H_2O$ | $H_2$ in Palladium | Mercury in Silver | Copper in nickel |

*Weak Hydrogen Bond*

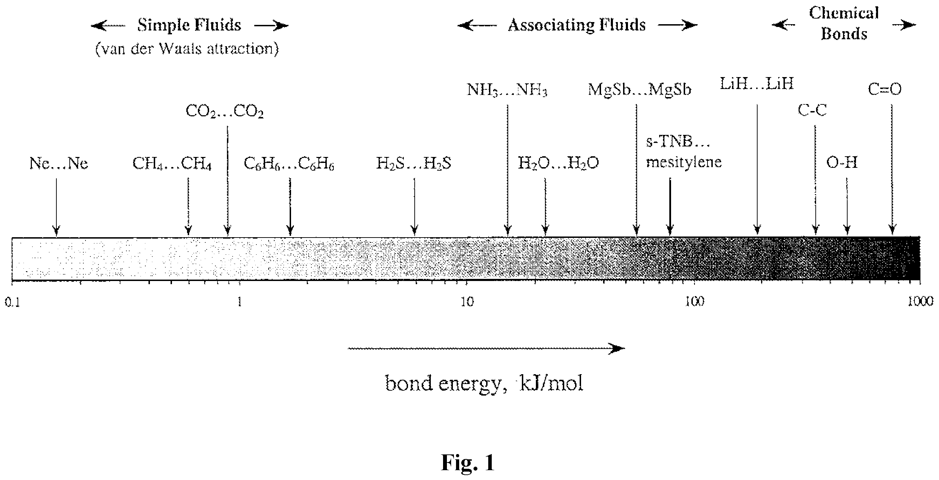

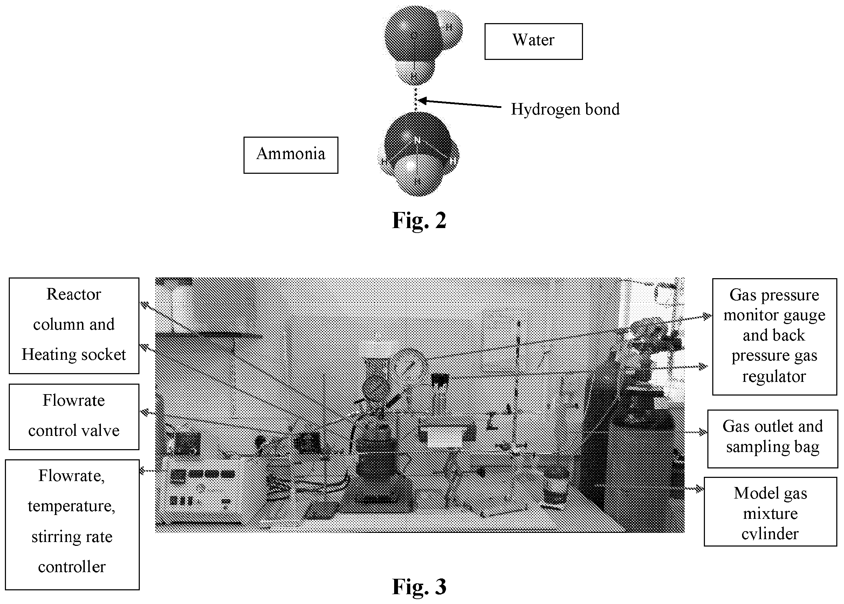

One of these intermolecular interaction called "hydrogen bond" is an attractive interaction between polar molecules in which hydrogen is bound to a highly electronegative atom. The bond energy range of a hydrogen bond is typically between 2 kJ/mol to 167 kJ/mol (Desiraju, G. R.; Steiner, T. The weak hydrogen bond: in structural chemistry and biology; Oxford University Press, 2001). Fig. 1 indicates the hydrogen bond (in so-called associating fluids) strength is intermediate between that of a strong chemical bond and the weak van der Waals interactions (Müller, E. A.; Gubbins, K. E. Industrial & Engineering Chemistry Research 2001, 40, 2193). The chemical bond typically range is from 63 kJ/mol to 1050 kJ/mol. On the other hand, van der Waals interactions, originating from the instantaneous attractive or dispersion forces between molecular entities, have energy normally less than 42 kJ/mol (Lee, L.-H. Fundamentals of adhesion; Springer, 1991). The typical hydrogen bond structure is X-H···A, where H is the hydrogen atom, X and A are the other atomic constituents. If X and A are highly electronegative, the deshielding of H and, in turn, the electrostatic attraction between H and A would be sufficiently strong to label the interaction as a hydrogen bond (Fig. 1 and 2) (Desiraju, G. R.; Steiner, T. The weak hydrogen bond: in structural chemistry and biology; Oxford University Press, 2001); it is noted that the bond strength is a significant evaluation criterion for this study.

To study the hydrogen bond and examine its bond strength, different techniques have been used. One important, accessible, technique is high resolution NMR; here the proton chemical shift is extremely sensitive to the existence of the H bond (Gerald, R.; Bernhard, T.; Haeberlen, U.; Rendell, J.; Opella, S. Journal of the American Chemical Society 1993, 115, 777). Hydrogen bonds are classified as very strong, strong and weak (Fig. 1) and Table 2 lists various properties of hydrogen bonds (Desiraju, G. R.; Steiner, T. The weak hydrogen bond: in structural chemistry and biology; Oxford University Press, 2001).

The hydrogen bond interactions between the light olefins and solvents are weaker than in the typical hydrogen bond. Nevertheless, only if such electrostatics interactions become weaker than van der Waals or dispersion forces (van Mourik, T.; van Duijneveldt, F. B. Journal of Molecular Structure: THEOCHEM 1995, 341, 63) do the hydrogen bonds fade into the so-called " van der Waals continuum" (Fig. 1).

**Table 2.** Properties of hydrogen bonds (Desiraju, G. R.; Steiner, T. The weak hydrogen bond: in structural chemistry and biology; Oxford University Press, 2001)

| Hydrogen bond | Very strong | strong | weak |
|---|---|---|---|
| Bond energy (kJ/mol) | 63 to 167 | 17 to 63 | < 17 |
| Bond Lengths (A) | 1.2 to 1.5 | 1.5 to 2.2 | 2.0 to 3.0 |
| $\theta$ Bond angle (X-H···A) range (° ) | 175 to 180 | 130 to 180 | 90 to 180 |
| IR $V_s$ relative shift* | 25% | 5% to 25% | < 5% |

(continued)

| Hydrogen bond | Very strong | strong | weak |
|---|---|---|---|
| **Examples** | [F···H···A]⁻ | O-H···O=C | C-H···O |
| | [N···H···N]⁺ | N-H···O = C | O-H···π |
| | P-OH···O=P | O-H···O-H | O-H···S |
| *define as V$_{IR}$= infrared spectrum relative shift. | | | |

*Molecular polarity*

The solvent employed in the process is normally a polar compound. Thus, considering the solubilises and selectivity of the light olefins and paraffins in various solvents (Prausnitz, J.; Anderson, R. AIChE Journal 1961, 7, 96) the polarities of molecules act as another important influence factor to influence the solubilisation processes.

Molecules are generally classified as being non-polar if they have zero dipole moment, and polar otherwise (Brinck, T.; Murray, J. S.; Politzer, P. Molecular Physics 1992, 76, 609). Molecular polarity refers to a separation of electric charge leading to a molecule or its chemical groups having an electric dipole or multipole moment (Brinck, T.; Murray, J. S.; Politzer, P. Molecular Physics 1992, 76, 609) and it depends on the difference in electronegativity between atoms in a molecule and the asymmetry of the molecular structure (Pritchard, H.; Skinner, H. Chemical Reviews 1955, 55, 745). Snyder (1987) concluded the polarity (index) of various compounds (Snyder, L. Journal of Chromatographic Science 1978, 16, 223; Snyder, L. R.; Kirkland, J. J.; Glajch, J. L. Practical HPLC method development; John Wiley & Sons, 2012). These polarity (index) parameters will be used in this research because they can affect the selection of the solvent applied in the extraction distillation process of the invention.

The polarity of the hydrocarbon molecule is increased by introduction of double bonds and more polar constituent groups (Maclay, W. N. Journal of Colloid Science 1956, 11, 272). In combination with the presence of weakly held electrons in the π-bond, these phenomena brings a weak and limited polarity to the olefins (Ahuja, S. Chromatography and Separation Science; Academic Press, 2003). Based on this theory and compare with the light paraffin's polarity, the polarity of the light olefin will be higher. Building upon the Prausnitz and Anderson theory, the hydrocarbon separation selectivity is sensitive to the polar solubility parameter (Lei, Z.; Li, C.; Chen, B. Separation & Purification Reviews 2003, 32, 121; Prausnitz, J.; Anderson, R. AIChE Journal 1961, 7, 96), and this parameter mainly dependent on the molecular polarity and molar volume (Prausnitz, J.; Anderson, R. AIChE Journal 1961, 7, 96). Scholars indicate that in order to have a much higher light olefin selectivity, the polar solubility parameter of the solvents should be as great as possible (Lei, Z.; Li, C.; Chen, B. Separation & Purification Reviews 2003, 32, 121; Prausnitz, J.; Anderson, R. AIChE Journal 1961, 7, 96). Specifically, a greater polar solubility parameter implies a larger polarity and smaller molar volume.

*Hildebrand and Hansen (Solubility) Parameters*

The Scatchard-Hildebrand theory is probably the most widely used solution theory (Kyle, B.; Leng, D. Industrial & Engineering Chemistry 1965, 57, 43). Its popularity is undoubtedly partly due to its simplicity, for it allows one to predict the thermodynamic properties of mixtures from readily available pure component properties (Kyle, B.; Leng, D. Industrial & Engineering Chemistry 1965, 57, 43).

The Scatchard-Hildebrand theory has two basic underlying assumptions (Kyle, B.; Leng, D. Industrial & Engineering Chemistry 1965, 57, 43):

a) The forces between molecules are dispersion forces (often called van der Waals' forces);
b) The entropy change on forming the solution has the same value as for an ideal solution.

Based on this theory Hildebrand proposed a definition for a "*solubility parameter*" that would provide a systemic description of the miscibility behavior of solvents. This solubility parameter is defined as the square root of the cohesive energy density, the heat of vaporization divided by the molar volume (Belmares, M.; Blanco, M.; Goddard, W.; Ross, R.; Caldwell, G.; Chou, S. H.; Pham, J.; Olofson, P.; Thomas, C. Journal of computational chemistry 2004, 25, 1814).

According to Hildebrand's solubility parameter approach (Reichardt, C.; Welton, T. Solvents and solvent effects in organic chemistry; John Wiley & Sons, 2011) two liquids are miscible if their solubility parameters $\delta$ differ by no more than ca. 7.0 MPa$^{1/2}$.[35] And mutual miscibility decreases as the $\delta$ values of two solvents become farther apart (Reichardt, C.; Welton, T. Solvents and solvent effects in organic chemistry; John Wiley & Sons, 2011). Higher mutual solubility will follow if the $\delta$ values of the solvents are closer.

There also exist another solubility parameters system, called Hansen solubility parameters. It was proposed by Hansen 1967 (Hansen, C. M. Danish Technical: Copenhagen 1967, 14). The basis of these solubility parameters is that the total energy of vaporization of a liquid consists of several individual parts (Hansen, C. M. Hansen solubility parameters: a user's handbook; CRC press, 2012). These arise from (atomic) dispersion forces, (molecular) permanent dipole-permanent dipole forces, and (molecular) hydrogen bonding (electron exchange) (Hansen, C. M. Hansen solubility parameters: a user's handbook; CRC press, 2012). That means the Hildebrand solubility parameter has been divided into these three dimensions by the Hansen solubility parameters (Hansen, C. M. Progress in Organic Coatings 2004, 51, 77).

Considering the three dimensions from the Hansen solubility parameters, the most general are the nonpolar interactions (Hansen, C. M. Hansen solubility parameters: a user's handbook; CRC press, 2012). These are derived from atomic force and have also been called *dispersion interactions* $\delta_d$ in the literature (Hansen, C. M. Hansen solubility parameters: a user's handbook; CRC press, 2012; Hansen, C. M. Progress in Organic Coatings 2004, 51, 77). The permanent dipole-permanent dipole interactions cause a second type of cohesion energy, the *polar cohesive energy* $\delta_p$. These are inherently molecular interactions and are found in most molecules to one extent or another. The dipole moment is primary parameter used to calculate these interactions. The third major cohesive energy source is hydrogen bonding, which can be called more generally an *electron exchange parameter* $\delta_h$. Hydrogen bonding is a molecular interaction and resembles the polar interactions in this respect. The basis of this type of cohesive energy is attraction among molecules because of the hydrogen bonds.

The relation between the Hildebrand and Hansen solubility parameters can be described by this equation (Hansen, C. M. Hansen solubility parameters: a user's handbook; CRC press, 2012; Hansen, C. M. Progress in Organic Coatings 2004, 51, 77):

$$\delta^2 = (\delta_d)^2 + (\delta_p)^2 + (\delta_h)^2$$

Here, $\delta$ is the Hildebrand solubility parameter, $\delta_d$ is the nonpolar interactions (*dispersion interactions*) of the Hansen solubility parameter, $\delta_p$ is the dipole-permanent dipole interactions (*polar cohesive energy*) of the Hansen solubility parameter, $\delta_h$ is the hydrogen bonding interactions (*electron exchange parameter*) of the Hansen solubility parameter. A comprehensive collection of Hildebrand and Hansen solubility parameters have been given by Barton (Reichardt, C.; Welton, T. Solvents and solvent effects in organic chemistry; John Wiley & Sons, 2011; Barton, A. F. CRC handbook of solubility parameters and other cohesion parameters; CRC press, 1991). In general, both of the Hildebrand and Hansen solubility parameters are closely bound up with the solubilisation and dissolution properties of the various organic solvents. Moreover, because the potential effect of these two parameters on selection of the solvent applied in the process of the invention, Hildebrand and Hansen solubility parameters also considered in this research.

*Temperature and pressure influences*

Lewis and Whitman (1924) indicate that, changes in temperature affect several factors, and it is necessary to distinguish clearly between the effect of temperature on the equilibrium and its effect on the coefficients of diffusion (Lewis, W.; Whitman, W. Industrial & Engineering Chemistry 1924, 16, 1215). Increase in temperature makes the gas less soluble, thus tending to lower the rate of absorption. The diffusion coefficients themselves may, however, be either raised or lowered by temperature, depending upon changes in film thickness and in specific diffusivity.

Since 1940 so much good gas solubility work at high pressure has appeared and the understanding of gas solubility of both low and high pressures has so improved that references to both low- and high-pressure solubilities are included (Battino, R.; Clever, H. L. Chemical Reviews 1966, 66, 395). Considering the pressure influence, Henry's law is used to quantify the solubility of gases in solvents (Lewis, W.; Whitman, W. Industrial & Engineering Chemistry 1924, 16, 1215; Battino, R.; Clever, H. L. Chemical Reviews 1966, 66, 395; Frolich, P. K.; Tauch, E. J.; Hogan, J. J.; Peer, A. A. Industrial & Engineering Chemistry 1931, 23, 548). The solubility of a gas in a solvent is directly proportional to the partial pressure of that gas above the solvent. Henry's law can be described as:

$$p = k_{\mathrm{H}} c$$

where $k_{\mathrm{H}}$ is a temperature-dependent constant, *p* is the partial pressure (atm), and *c* is the concentration of the dissolved gas in the liquid (mol/L). This relationship indicates that, under constant temperature the increasing of the gas partial pressure leads higher concentration of the dissolved gas in the liquid (Frolich, P. K.; Tauch, E. J.; Hogan, J. J.; Peer, A. A. Industrial & Engineering Chemistry 1931, 23, 548).

### Experimental

***Preparation of solvents, Model Catalytic Cracking and Thermal Cracking Gas Mixture (MCCGMIMTCGM)***

The various solvents, methanol, n-hexane and dimethyl sulfoxide (DMSO) with stated purity of 99.9%, propylene carbonate (PC), dimethyl carbonate (DMC) with purity of 99%, methanol-$d_4$ with purity of 99.8%, n-hexane-$d_{14}$ with purity of 99% and dimethyl sulfoxide-$d_6$ with purity of 99.6% were all purchased from Sigma Aldrich Company. Based on the literature, it was found that there are two types of petroleum pyrolysis gas; catalytic cracking gas and thermal cracking gas (Safarik, D. J.; Eldridge, R. B. Industrial & Engineering Chemistry Research 1998, 37, 2571; CNCIC "Gas Market Research Report of China," China National Chemical Information Center, 2012). These gas mixtures are produced from refineries' catalytic cracking process and thermal cracking process, respectively (CNCIC "Gas Market Research Report of China," China National Chemical Information Center, 2012).

In the research described herein, model catalytic cracking gas mixture (MCCGM) contains 14.97% ethylene, 24.97% ethane, 19.87% propylene and 40.19% propane in mol%. The components reference of the MCCGM is from the Gas Market Research Report of China (2011) (CNCIC "Gas Market Research Report of China," China National Chemical Information Center, 2012). According to another report, the components of the model thermal cracking gas mixture (MTCGM) used herein were chosen (Safarik, D. J.; Eldridge, R. B. Industrial & Engineering Chemistry Research 1998, 37, 2571). This gas mixture contained 15.00% hydrogen, 4.01% carbon monoxide, 24.76% methane, 45.21% ethylene, 4.99% ethane, 5.02% propylene and 1.01% propane in mol%.

***Methods***

*Separation Analysis using Gas Chromatography Spectrometry (GC)*

The separation experiment involved careful mixing and separation methods. Five 280ml solvent samples were prepared, the components in each sample and their volume ratio are (i)* n-hexane and DMC, 1:1; (ii)* pure DMC; (iii) methanol and DMC 1:1; (iv)* propylene and carbonate, 1:1; and (v)* DMSO and DMC, 1:1, respectively and subsequently labeled (i), (ii) etc. These solvent samples had been doubled, and then applied in the MCCGM separation (i to v) or MTCGM separation (vi to x) processes. And these solvent mixtures are confirmed to be homogeneous under the experiment conditions. *Solvent samples (i), (ii), (iv) and (v), and (vi), (vii), (ix) and (x) are not part of the invention.

The separation process was processed using the Parr 4560 Mini Bench Top Reactor. The picture of the separation equipment's detail structure is displayed (Fig. 3). Each 280ml solvent was poured into the reactor column when they prepared. Then the reactor column was installed and the air tightness had been checked. After all the equipments were checked and ready, the experiment was launched. Two major steps had been processed in the experiment. One is the adsorption process, the other one is the desorption process.

During the adsorption process, the pressure in the reactor was increased with constant gas flow (10ml/min) from the model gas mixture cylinder until a pressure of 2 bar above atmospheric pressure (i.e. a guage pressure of 2 bar) was reached. The stirrer was kept rotating to ensure maximal adsorption of the model gas mixture. Samples were collected and stored by the syringes under different pressures from the gas outlet of the reactor.

In the desorption step, the gas inlet had been switched off. The pressure in the reactor which obtained during the adsorption was released step by step. In each step, 1 bar was released, and the stirrer was left to run for 30 minutes to ensure that the system equilibrated (i.e. released all the adsorbed gas) under that particular pressure. Thus, in a first step, the pressure was reduced from 2 bar above atmospheric pressure, down to 1 bar above atmospheric pressure, and the the stirrer was left to run for 30 minutes for the system to equilibrate at the pressure of 1 bar above atmospheric. Then, in a second step, the pressure was reduced from 1 bar above atmospheric pressure, down to atmospheric pressure, and the stirrer was left to run for 30 minutes at atmospheric pressure, for the system to equilibrate at atmospheric pressure. Gases released under these different pressures (i.e. at 1 bar above atmospheric pressure, and then at atmospheric pressure) were collected and stored in numbered gas sampling bags. After the gas at all these pressures had been released, the reactor was heated periodically until the temperature in the reactor reached a certain value (normally close to the lower boiling point of the solvent in the mixture). The stirrer was kept running for 30 minutes at each temperature stage researched, to ensure gas desorption. Gases released during the heating process were also collected and stored in numbered gas sampling bags.

The same progress (except the heat process) was repeated to test the gas separation performance of pure methanol under various temperatures (-21 °C, room temperature/25 °C and 40 °C).

Following the separation experiment, GC method was employed to quantify the different contents in the collected gas samples. Thereupon, the collected gas samples of (i) to (x) solvents were measured successively. The GC analyser was a SHIMADZU GC2014 gas chromatography spectrometer. The main operating parameters of GC meter were: Column Oven Temperature 45 °C; Injection Temperature 300 °C; At 45 °C system held 4 minutes, then temperature rising rate from 45 to 200 °C was 50 °C /min, and after 200 °C system held for another 7.8 minutes, the temperature rising rate was changed to 10 °C /min, until it reached 300 °C.

***Calculations***

In this research, a new calculation system for evaluating the olefins selectively/olefins and paraffins separation performance by applying various solvents has been set up.

$$ S_i = \frac{m_{pi}}{m_{oi}} $$

where $S_i$ *(i=2,3)* is the different solvent's C2 or C3 olefins selectively (olefins and paraffins separation performance), $m_{pi}$ *(i=2,3)* is the molar percentage (mol%) of the C2 and C3 paraffin, $m_{oi}$ *(i=2,3)* is the molar percentage (mol%) of the C2 and C3 olefin, respectively. Based on the molar fraction of different contents in the MCCGM/MTCGM, two individual evaluation systems, respectively, were constructed.

1) For the MCCGM:

The ethane/ethylene molar ratio in the original MCCGM is 1.67.
The propane/propylene molar ratio in the original MCCGM is 2.02.
If the value of C2 olefin selectively $S_2$ is higer than 1.67 at adsorption stage and lower than 1.67 at desorption (highest temperature) stage of the extractive distillation, it indicates the light olefins have been concentrated through the process of the invention. This means the solvent can successfully separate the C2 olefin and paraffin contents in the MCCGM. Otherwise, the solvent is defined as noneffective. It is similar in the C3 olefin selectively calculation. If $S_3$ is higer than 2.02 at adsorption stage and lower than 2.02 at desorption stage, it means solvent can successfully separate the C3 olefin and paraffin contents in the MCCGM.

2) For the MTCGM:

The ethane/ethylene molar ratio in the original MTCGM is 0.11.
The propane/propylene molar ratio in the original MTCGM is 0.20.
Except the different reference value of C2 and C3 olefin selectively, all the evaluation processes are identical to the processes of the MCCGM separation.

***Results and discussions***

*MCCGM separation*

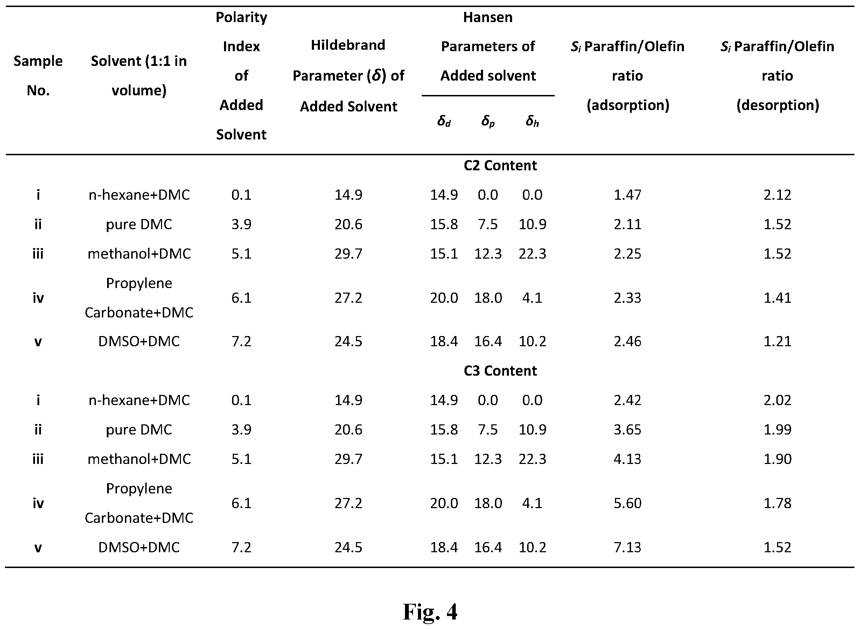

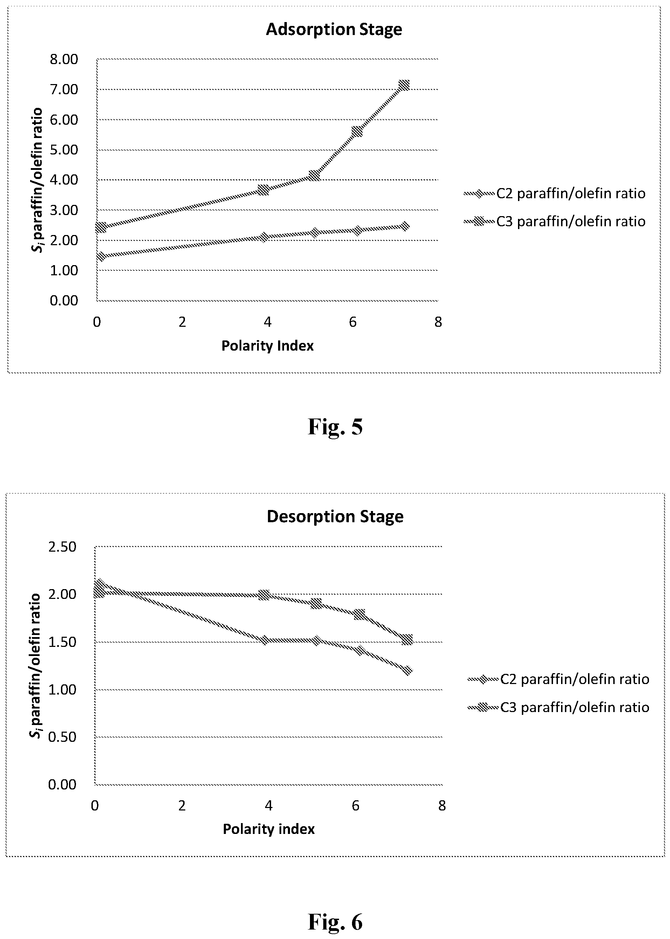

Solvent samples with number (i) to (v) were applied in the extractive distillation process of the invention* to test their feasibility for separating the olefin and paraffin content in the MCCGM (*solvent sample (iii) is part of the invention and solvent samples (i), (ii), (iv) and (v) are not part of the invention). The separation performance $S_i$ results are indicated in the table in Fig. 4.
Because almost all organic solvents are miscible with DMC, DMC was used as a basis solvent. In order to examine the polarity, Hildebrand and Hansen parameters effect to the separation performance, except sample (ii), various solvents were added in to the DMC with 1:1 volume ratio in all the other samples. The polarities, Hildebrand and Hansen parameters of the various added organic solvents are displayed in the table in Fig. 4. Compared with the pure DMC as the separation solvent in sample (ii), the differences of the added solvents' polarity in other samples lead some changes which reflect in their separation performance, and these changes do have trends. Based on the results data in Fig. 4, the graphs in Figs. 5 and 6 have been drawn up for easier understanding of the polarity effect and the trends.
Fig. 5 indicates that in the adsorption stage of the extractive distillation process, both the C2 and C3 paraffin/olefin ratio in the gas outlet stream are increasing with the rising value of the added solvent polarity. That means the olefin content is adsorbed more into the solvent phase with higher solvent polarity. Moreover, except sample (i) the n-hexane and DMC mixture, which get the lowest polarity among (i) to (v) samples, all the other samples achieve higher paraffin/olefin ratio than the original paraffin/olefin ratio in the MCCGM. Therefore, sample (ii) to (v)'s separation feasibilities are initially proved in the adsorption stage.
Through the desorption results displayed in the Fig. 6 the C2 and C3 paraffin/olefin ratio in the gas outlet stream present negative correlation with the value of the added solvent polarity. It indicates that, because with higher polarity solvent, more olefin content adsorbed in the adsorption stage. Thus, during desorption solvent with higher polarity discharges more olefin to the gas outlet stream. Based on the previous evaluation method, solvents (ii) to (v) are confirmed to have the feasibility for separating the light olefin and paraffin content in our extraction distillation process (NB. solvent sample (iii) is part of the invention and solvent samples (i), (ii), (iv) and (v) are not part of the invention).

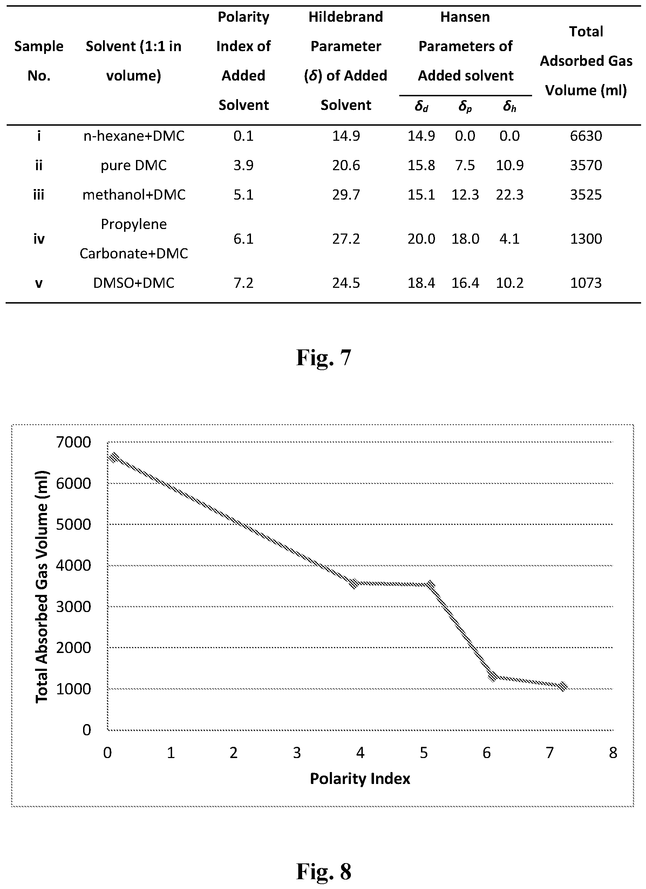

The separation performance affect by the solvent polarity can be explained through the Prausnitz and Anderson theory (Prausnitz, J.; Anderson, R. AIChE Journal 1961, 7, 96). The theory indicates the hydrocarbon separation selectivity depend on the solvent molecular polarity and molar volume (Lei, Z.; Li, C.; Chen, B. Separation & Purification Reviews 2003, 32, 121; Prausnitz, J.; Anderson, R. AIChE Journal 1961, 7, 96). With larger polarity the solvent will have higher light olefin selectivity. Based on Hildebrand parameter's definition, which indicates higher mutual solubility will follow if the $\delta$ values of the solvents are closer (Reichardt, C.; Welton, T. Solvents and solvent effects in organic chemistry; John Wiley & Sons, 2011). The total volume of MCCGM adsorbed by the various solvent is considered (table in Fig. 7).

In these results, the total adsorbed gas volume presents a negative trend when it considered with the increasing polarity. One point we can confirm is that, when the solvent has the lowest polarity, Hildebrand and Hansen (all three dimensions) parameters, it can absorb the largest volume of the MCCGM.

The various pressure settings also have been introduced into the experiment's adsorption stage to examine the pressure effect to the separation performance. From the recorded data about paraffin/olefin ratio in the gas outlet stream under various pressures, it shows this ratio present a negative correlation with the increasing pressure in the reactor. This phenomenal can be determine as the result of the pressure effect. Because Henry's law indicates:

$$p = k_{\mathrm{H}} c$$

where $k_{\mathrm{H}}$ is a temperature-dependent constant, $p$ is the partial pressure (atm), and $c$ is the concentration of the dissolved gas in the liquid (mol/L) (Lewis, W.; Whitman, W. Industrial & Engineering Chemistry 1924, 16, 1215; Battino, R.; Clever, H. L. Chemical Reviews 1966, 66, 395).

Therefore, when the temperature in the reactor is constant and the total pressure is rising up. The partial pressure of both olefin and paraffin content are inclined. In the MCCGM, both the C2 and C3 paraffin have higher concentration than the olefin content. Thus, with higher pressure, paraffins' partial pressure change is bigger than olefins' (Lewis, W.; Whitman, W. Industrial & Engineering Chemistry 1924, 16, 1215; Battino, R.; Clever, H. L. Chemical Reviews 1966, 66, 395). According to Henry's law, it is confirmed that, at higher pressure more paraffin content dissolved into the solvent phase than the olefin content (Lewis, W.; Whitman, W. Industrial & Engineering Chemistry 1924, 16, 1215; Battino, R.; Clever, H. L. Chemical Reviews 1966, 66, 395). Hence, the paraffin/olefin ratio $S_i$ is decreased. This means the olefins and paraffins separation performance is moving towards lower efficiency with the higher pressures.

*Temperature effect on MCCGM separation*

Besides the pressure, the temperature effect was also investigated in this study. Table 3 presents the pure methanol separation performance at different temperatures and constant pressure. The results clearly indicate that, the increasing temperature leads the C2 and C3 contents' S, value dropped during the adsorption stage. The reason is increase in temperature makes the gas less soluble, thus tending to lower the rate of absorption (Lewis, W.; Whitman, W. Industrial & Engineering Chemistry 1924, 16, 1215). Also because the partial pressures of the paraffins are higher than the olefins'. Based on Henry's law, compare with the low temperature situation, methanol absorbs more paraffins at high temperature than olefins (Lewis, W.; Whitman, W. Industrial & Engineering Chemistry 1924, 16, 1215; Battino, R.; Clever, H. L. Chemical Reviews 1966, 66, 395). Therefore, during the desorption stage, the higher the operation temperature is, the bigger the paraffin/olefin ratio is observed from the gas outlet stream. From this point of view, it can be confirmed that the separation performance has negative correlation with the operation temperature.

**Table 3.** Pure methanol separation performance at different temperature with MCCGM.

| Solvent and Temperature | $S_i$ Paraffin/Olefin ratio (adsorption) | $S_i$ Paraffin/Olefin ratio (desorption) |
|---|---|---|
| **C2 Content** | | |
| **-18**°C | 2.06 | 1.60 |
| **25**°C | 1.87 | 1.67 |
| **40**°C | 1.75 | 1.70 |

(continued)

| C3 Content | | |
|---|---|---|
| **-18**°C | 3.10 | 1.78 |
| **25**°C | 3.04 | 1.85 |
| **40**°C | 3.00 | 1.88 |

*MTCGM separation*

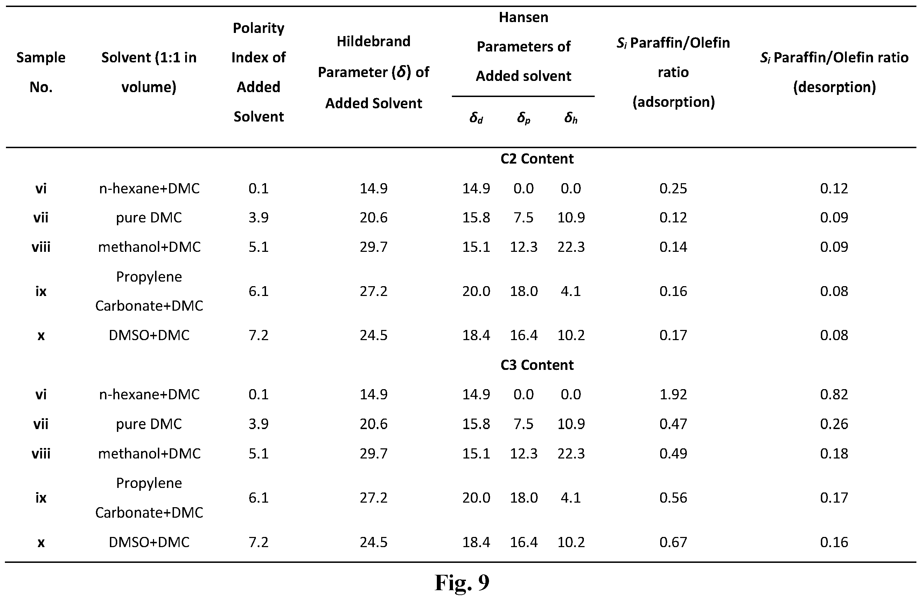

Similarly, sample (vi) to (x) were also applied to test their separation performance of MTCGM. Their separation results were also calculated and indicated in the table in Fig. 9. NB. solvent samples (vi), (vii), (ix) and (x) are not part of the invention.

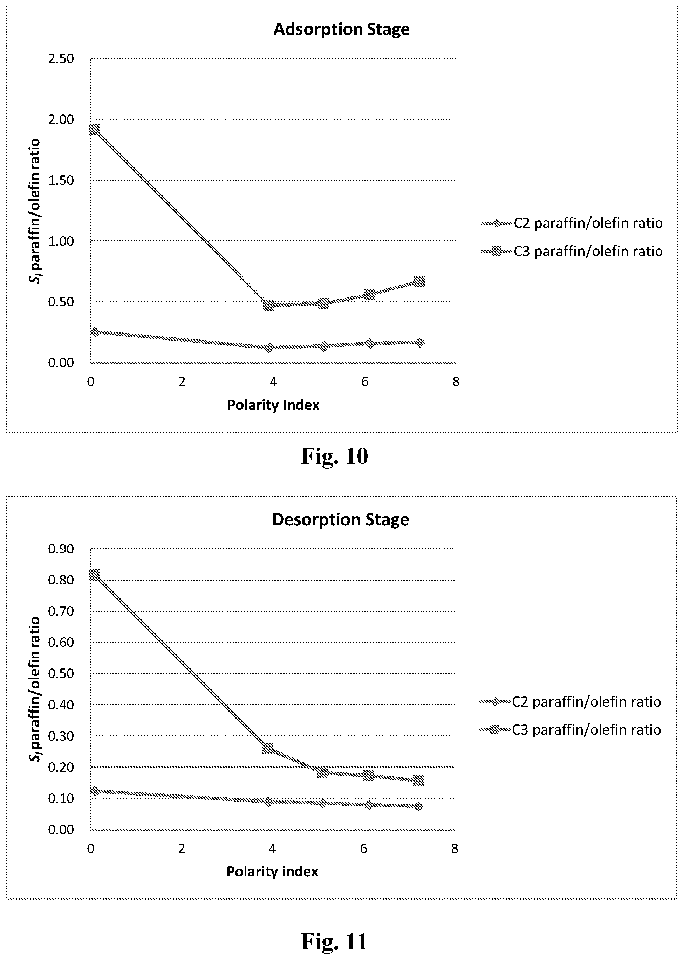

According to the results data presented in the table 6 in Fig. 9, Figs. 10 and 11 have been constructed. It can be observed from Fig.10, sample (vi)'s $S_i$ value during the adsorption stage is relatively higher than other samples. This is because compared with other solvents, the Hildebrand parameter and Hansen parameters $\delta_p/\delta_h$ of n-hexane and gas contents are closer (Reichardt, C.; Welton, T. Solvents and solvent effects in organic chemistry; John Wiley & Sons, 2011; Hansen, C. M. Hansen solubility parameters: a user's handbook; CRC press, 2012; Barton, A. F. CRC handbook of solubility parameters and other cohesion parameters; CRC press, 1991). In MTCGM the partial pressure of the olefins are higher than paraffins'. Therefore, based on Hildebrand, Hansen and Henry's theory (Kyle, B.; Leng, D. Industrial & Engineering Chemistry 1965, 57, 43; Hansen, C. M. Hansen solubility parameters: a user's handbook; CRC press, 2012; Lewis, W.; Whitman, W. Industrial & Engineering Chemistry 1924, 16, 1215; Battino, R.; Clever, H. L. Chemical Reviews 1966, 66, 395) more light olefins have been absorbed by sample (vi) than other samples.

Considering other samples, it indicates that in the adsorption stage of the extractive distillation process, both the C2 and C3 paraffin/olefin ratio in the gas outlet stream are increasing with the rising value of the added solvent polarity. This trend is the same as in the MCCGM test, which means the absorbance of light olefins has a positive correlation with the solvent polarity. Moreover, all the samples observed higher paraffin/olefin ratio than the original paraffin/olefin ratio in the MTCGM. Therefore, light olefins can be successfully absorbed by sample (vi) to (x) in the adsorption stage.

The desorption results displayed in the Fig. 11 shows the C2 and C3 paraffin/olefin ratio in the gas outlet stream present negative correlation with increasing solvent polarity. Sample (vi) to (x) are confirmed to have the feasibility for separating the C2 olefin and paraffin content; sample (vii) to (x) are confirmed to have the feasibility for separating the C3 olefin and paraffin content as the reason of their paraffin/olefin ratio $S_i$ are lower than 0.11 and 0.20, respectively. The cause of these differences in the separation performance affect by the solvent polarity already explained in the MCCGM separation part.

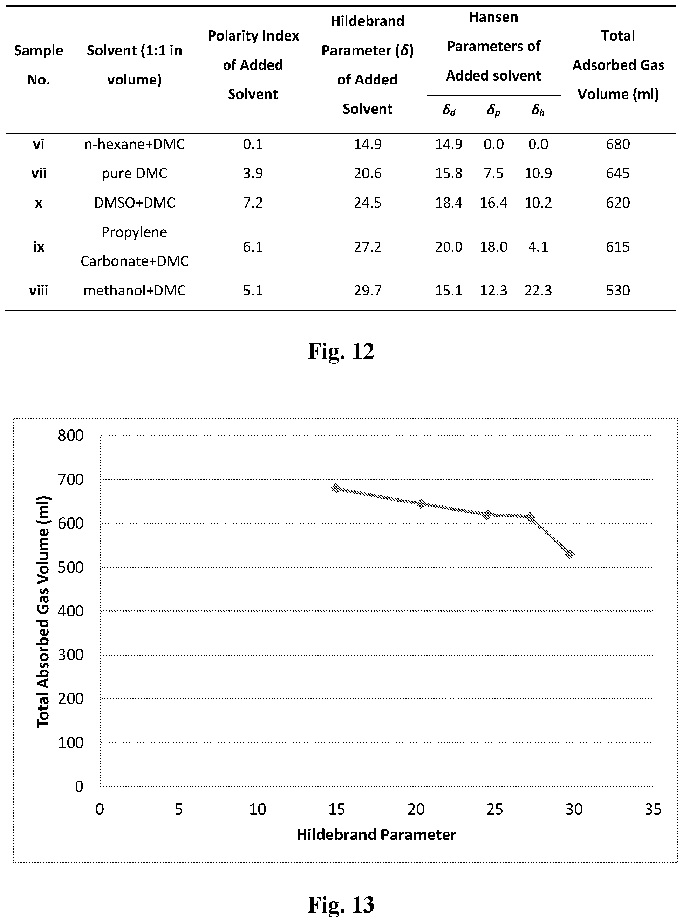

The total volume of MTCGM adsorbed by the various solvent was then also considered (see the table in Fig. 12). The total adsorbed gas volume presents a negative trend when it considered with the increasing value of the Hildebrand parameter (Fig. 13). Compared with the solvents' polarity the total adsorbed gas volume presents a relatively clear negative trend (except the methanol+DMC sample) when it is considered with the increasing value of the Hansen dispersion interactions parameter $\delta d$. Whilst, the polar cohesive energy $\delta p$ of the Hansen parameters also shows a negative correlation with the total adsorbed gas volume (except the methanol+DMC sample). Just like the MCCGM case, the solvent with the lowest polarity, Hildebrand and Hansen parameters can absorb the largest volume of the MTCGM.

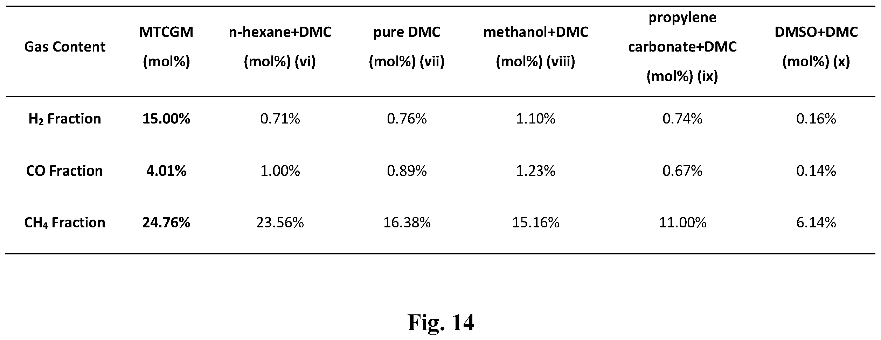

Moreover, except C2/C3 contents there are three kinds of other gases exist in the MTCGM, which are hydrogen, monoxide and methane. Therefore, in order to further confirm the feasibility of the extractive distillation process applied in the thermal cracked gas separation, the fraction change of these gas contents also need to be monitored. The table in Fig. 14 gives the molar fraction of hydrogen, monoxide and methane in the original MTCGM and each sample's outlet gas in the desorption stage, respectively. The molar fraction of hydrogen, monoxide and methane in all the samples are reduced, meaning that all five samples can remove these gas contents from MTCGM successfully. With the increasing solvent polarity, the separation process presents a positive correlation of the hydrogen, monoxide and methane reduction performance. The best performance is achieved when the highest polarity mixture of DMSO with DMC is applied as the solvent in the extractive distillation process.

As the MCCGM experiment, to examine the pressure effect to the separation performance, various pressure settings were applied in the MTCGM experiment's adsorption stage. From the recorded data about paraffin/olefin ratio in the gas outlet stream under various pressures, it shows this ratio present a negative correlation with the increasing pressure in the reactor. This phenomenon has been explained hereinbefore.

*Conclusions*

Through this series of tests the feasibility and effectiveness of applying various organic solvents as the extractant in the cracked gas separation process was investigated. The analysis data represent very positive results.

Upon analysing the results from all the tests, it can be concluded that, polarity has a very clear and accurate relationship with the olefin/paraffin separation performance. Solvents with relatively high polarity provide improved performances than lower polarity mixtures. On the other hand, in the MTCGM experiment the solvents' Hildebrand solubility parameters present a negative correlation with the total adsorbed gas volume. It is also confirmed that the separation performance has a negative correlation with the operation temperature.

Based on these conclusions, it could be proposed that the ideal extractant of the gas separation process should have relatively high polarity, and a relatively low Hildebrand solubility parameter. Therefore, with the advantages of efficient olefin/paraffin separation ratio and high total adsorbed gas volume in MTCGM experiment, the solvent DMSO exhibited relatively better performance than all the other solvents in the cracked gas separation process.

**Claims**

1. A process for separating one or more hydrocarbons from a mixture of hydrocarbons, wherein the mixture of hydrocarbons is a composition which comprises a mixture of two or more hydrocarbons that are in the gas state at 298.15 K and 100,000 Pa, which mixture comprises:

   (a) one or more first hydrocarbons, and
   (b) one or more further hydrocarbons which are other than the one or more first hydrocarbons,

   wherein the one or more first hydrocarbons comprise one or more alkenes, and the one or more further hydrocarbons comprise one or more alkanes,
   which process comprises:

   contacting the mixture of hydrocarbons with an extractant suitable for extracting the one or more first hydrocarbons, which extractant comprises methanol, in an amount of equal to or greater than 40 wt%; and thereby forming:

   (i) a first phase comprising the extractant and the one or more first hydrocarbons; and
   (ii) a second phase comprising said mixture having a reduced content of the one or more first hydrocarbons.

2. A process according to claim 1 wherein the one or more alkenes are selected from $C_{2-4}$ alkenes and the one or more alkanes are selected from $C_{1-4}$ alkanes.

3. A process according to claim 1 or claim 2 wherein the one or more alkenes comprise ethylene and propylene and the one or more alkanes comprise ethane and propane.

4. A process according to any one of the preceding claims wherein the mixture is obtainable by catalytic cracking of petroleum.

5. A process according to claim 1 or claim 2 wherein the one or more alkenes comprise ethylene and propylene and the one or more alkanes comprise methane, ethane and propane.

6. A process according to any one of claims 1 to 3 and 5 wherein the mixture comprises hydrogen, carbon monoxide, methane, ethylene, ethane, propylene and propane, optionally wherein the mixture further comprises an inert gas, optionally wherein the inert gas comprises nitrogen.

7. A process according to any one of claims 1 to 3, 5 and 6 wherein the mixture is obtainable by thermal cracking of petroleum.

8. A process according to any one of claims 1 to 3, 5 and 6 wherein the mixture is natural gas, shale gas, coal bed gas, biogas, refinery gas, or a gas which is obtainable by catalytic and/or thermal cracking of petroleum.

9. A process according to any one of the preceding claims wherein the mixture further comprises one or more organosulfur compounds, the first phase further comprises the one or more organosulfur compounds, and the second phase has a reduced content of the one or more organosulfur compounds.

10. A process according to any one of the preceding claims wherein the extractant comprises said methanol in an amount of equal to or greater than 50 wt%.

11. A process according to any one of the preceding claims wherein the extractant comprises said methanol which is a first polar solvent, and a second solvent which is different from said first polar solvent and is miscible with the first polar solvent, wherein the second solvent is a second polar solvent, and wherein the first and second polar solvents are different polar organic solvents, and wherein each of the first and second solvents has a Hildebrand solubility parameter ($\delta$) which is equal to or greater than 18 MPa$^{1/2}$ and a polarity index which is equal to or greater than 3.

12. A process according to any one of the preceding claims wherein the extractant comprises said methanol and dimethylcarbonate (DMC).

13. A process according to any one of the preceding claims wherein the contacting is performed:

at a first temperature, which first temperature is from -30 °C to 60 °C and is optionally from 5 °C to 35 °C; and
at a first pressure, which first pressure is greater than 1 bar and is optionally greater than 1 bar and less than or equal to 300 bar.

14. A process according to any one of the preceding claims which further comprises recovering the mixture having a reduced content of the one or more first hydrocarbons, optionally wherein recovering said mixture having a reduced content of the one or more first hydrocarbons comprises separating the second phase from the first phase.

15. A process according to any one of the preceding claims which further comprises recovering the one or more first hydrocarbons from the first phase wherein, after recovering the one or more first hydrocarbons from the first phase, the process optionally further comprises contacting the second phase with the extractant from the first phase, and thereby forming:

(i) a further first phase comprising the extractant and said one or more first hydrocarbons; and
(ii) a further second phase comprising said mixture having a further reduced content of the one or more first hydrocarbons;
optionally wherein the process further comprises:

(a) recovering the one or more first hydrocarbons from the further first phase wherein, after recovering the one or more first hydrocarbons from the further first phase, the process optionally further comprises:
(b) contacting the further second phase with the extractant from the further first phase, and thereby forming:
(i) a further first phase comprising the extractant and said one or more first hydrocarbons, and (ii) a further second phase comprising said mixture having a further reduced content of the one or more first hydrocarbons; and
(c) optionally repeating steps (a) and (b) one or more times.

**Patentansprüche**

1. Verfahren zur Abtrennung von einem oder mehreren Kohlenwasserstoffen aus einem Kohlenwasserstoffgemisch, wobei das Kohlenwasserstoffgemisch eine Zusammensetzung ist, die ein Gemisch aus zwei oder mehr Kohlenwasserstoffen umfasst, die sich bei 298,15 K und 100.000 Pa im Gaszustand befinden, wobei das Gemisch Folgendes umfasst:

(a) einen oder mehrere erste Kohlenwasserstoffe, und
(b) einen oder mehrere weitere Kohlenwasserstoffe, die von dem einen oder den mehreren ersten Kohlenwasserstoffen verschieden sind,

wobei der eine oder die mehreren ersten Kohlenwasserstoffe ein oder mehrere Alkene umfassen, und der eine oder die mehreren weiteren Kohlenwasserstoffe ein oder mehrere Alkane umfassen,

wobei das Verfahren Folgendes umfasst:

Inkontaktbringen des Kohlenwasserstoffgemisches mit einem Extraktionsmittel, das zur Extraktion des einen oder der mehreren ersten Kohlenwasserstoffe geeignet ist, wobei das Extraktionsmittel Methanol in einer Menge von gleich oder mehr als 40 Gew.-% umfasst; und dadurch Bilden:

(i) einer ersten Phase, die das Extraktionsmittel und den einen oder die mehreren ersten Kohlenwasserstoffe umfasst; und
(ii) einer zweiten Phase, die das Gemisch mit einem reduzierten Gehalt an dem einen oder den mehreren ersten Kohlenwasserstoffen umfasst.

2. Verfahren nach Anspruch 1, wobei das eine oder die mehreren Alkene ausgewählt sind aus $C_{2-4}$-Alkenen und das eine oder die mehreren Alkane ausgewählt sind aus $C_{1-4}$-Alkanen.

3. Verfahren nach Anspruch 1 oder Anspruch 2, wobei das eine oder die mehreren Alkene Ethylen und Propylen umfassen und das eine oder die mehreren Alkane Ethan und Propan umfassen.

4. Verfahren nach einem der vorhergehenden Ansprüche, wobei das Gemisch durch katalytisches Cracken von Erdöl erhältlich ist.

5. Verfahren nach Anspruch 1 oder Anspruch 2, wobei das eine oder die mehreren Alkene Ethylen und Propylen umfassen und das eine oder die mehreren Alkane Methan, Ethan und Propan umfassen.

6. Verfahren nach einem der Ansprüche 1 bis 3 und 5, wobei das Gemisch Wasserstoff, Kohlenmonoxid, Methan, Ethylen, Ethan, Propylen und Propan umfasst, wobei das Gemisch optional ferner ein Inertgas umfasst, wobei das Inertgas optional Stickstoff umfasst.

7. Verfahren nach einem der Ansprüche 1 bis 3, 5 und 6, wobei das Gemisch durch thermisches Cracken von Erdöl erhältlich ist.

8. Verfahren nach einem der Ansprüche 1 bis 3, 5 und 6, wobei das Gemisch Erdgas, Schiefergas, Kohleflözgas, Biogas, Raffineriegas oder ein Gas ist, das durch katalytisches und/oder thermisches Cracken von Erdöl gewonnen werden kann.

9. Verfahren nach einem der vorhergehenden Ansprüche, wobei das Gemisch ferner eine oder mehrere schwefelorganische Verbindungen enthält, die erste Phase ferner die eine oder mehreren schwefelorganischen Verbindungen enthält, und die zweite Phase einen reduzierten Gehalt an der einen oder den mehreren schwefelorganischen Verbindungen aufweist.

10. Verfahren nach einem der vorhergehenden Ansprüche, wobei das Extraktionsmittel das Methanol in einer Menge von gleich oder mehr als 50 Gew.-% enthält.

11. Verfahren nach einem der vorhergehenden Ansprüche, wobei das Extraktionsmittel das Methanol, das ein erstes polares Lösungsmittel ist, und ein zweites Lösungsmittel, das sich von dem ersten polaren Lösungsmittel unterscheidet und mit dem ersten polaren Lösungsmittel mischbar ist, umfasst, wobei das zweite Lösungsmittel ein zweites polares Lösungsmittel ist, und wobei das erste und das zweite polare Lösungsmittel unterschiedliche polare organische Lösungsmittel sind, und wobei jedes der ersten und zweiten Lösungsmittel einen Hildebrand-Löslichkeitsparameter ($\delta$), der gleich oder größer als 18 MPa$^{1/2}$ ist, und einen Polaritätsindex, der gleich oder größer als 3 ist, aufweist.

12. Verfahren nach einem der vorhergehenden Ansprüche, wobei das Extraktionsmittel Methanol und Dimethylcarbonat (DMC) umfasst.

13. Verfahren nach einem der vorhergehenden Ansprüche, wobei das Inkontaktbringen durchgeführt wird:

bei einer ersten Temperatur, wobei die erste Temperatur von -30 °C bis 60 °C und optional von 5 °C bis 35 °C beträgt; und

bei einem ersten Druck, wobei der erste Druck größer als 1 bar ist und optional größer als 1 bar und kleiner oder gleich 300 bar ist.

**14.** Verfahren nach einem der vorhergehenden Ansprüche, das ferner die Gewinnung des Gemisches mit einem reduzierten Gehalt an dem einen oder den mehreren ersten Kohlenwasserstoffen umfasst, wobei die Gewinnung des Gemisches mit einem reduzierten Gehalt an dem einen oder den mehreren ersten Kohlenwasserstoffen optional die Abtrennung der zweiten Phase von der ersten Phase umfasst.

**15.** Verfahren nach einem der vorhergehenden Ansprüche, das ferner die Gewinnung des einen oder der mehreren ersten Kohlenwasserstoffe aus der ersten Phase umfasst, wobei das Verfahren nach der Gewinnung des einen oder der mehreren ersten Kohlenwasserstoffe aus der ersten Phase optional ferner das Inkontaktbringen der zweiten Phase mit dem Extraktionsmittel aus der ersten Phase umfasst, wodurch Folgendes gebildet wird:

(i) eine weitere erste Phase, die das Extraktionsmittel und den einen oder die mehreren ersten Kohlenwasserstoffe umfasst; und
(ii) eine weitere zweite Phase, die das Gemisch mit einem weiter reduzierten Gehalt an dem einen oder den mehreren ersten Kohlenwasserstoffen umfasst;
wobei das Verfahren optional ferner Folgendes umfasst:

(a) Rückgewinnen des einen oder der mehreren ersten Kohlenwasserstoffe aus der weiteren ersten Phase, wobei das Verfahren nach der Gewinnung des einen oder der mehreren ersten Kohlenwasserstoffe aus der weiteren ersten Phase optional ferner Folgendes umfasst:
(b) Inkontaktbringen der weiteren zweiten Phase mit dem Extraktionsmittel aus der weiteren ersten Phase und dadurch Bilden: (i) einer weiteren ersten Phase, die das Extraktionsmittel und den einen oder die mehreren ersten Kohlenwasserstoffe umfasst, und (ii) einer weiteren zweiten Phase, die das Gemisch mit einem weiter reduzierten Gehalt an dem einen oder den mehreren ersten Kohlenwasserstoffen umfasst; und
(c) optional ein- oder mehrmaliges Wiederholen der Schritte (a) und (b).

## Revendications

**1.** Procédé de séparation d'un ou plusieurs hydrocarbures d'un mélange d'hydrocarbures, dans lequel le mélange d'hydrocarbures est une composition qui comprend un mélange de deux ou plusieurs hydrocarbures à l'état gazeux à 298,15 K et 100000 Pa, lequel mélange comprend :

(a) un ou plusieurs premiers hydrocarbures, et
(b) un ou plusieurs autres hydrocarbures qui sont autres que l'un ou plusieurs premiers hydrocarbures,

dans lequel l'un ou plusieurs premiers hydrocarbures comprennent un ou plusieurs alcènes, et l'un ou plusieurs autres hydrocarbures comprennent un ou plusieurs alcanes,
lequel procédé comprend :

la mise en contact du mélange d'hydrocarbures avec un agent d'extraction adapté pour extraire l'un ou plusieurs premiers hydrocarbures, lequel agent d'extraction comprend du méthanol, en une quantité égale ou supérieure à 40 % en poids ;
et formant ainsi :

(i) une première phase comprenant l'agent d'extraction et l'un ou plusieurs premiers hydrocarbures ; et
(ii) une deuxième phase comprenant ledit mélange ayant une teneur réduite de l'un ou plusieurs premiers hydrocarbures.

**2.** Procédé selon la revendication 1, dans lequel l'un ou plusieurs alcènes sont choisis parmi les alcènes en $C_{2-4}$ et l'un ou plusieurs alcanes sont choisis parmi les alcanes en $C_{1-4}$.

**3.** Procédé selon la revendication 1 ou 2, dans lequel l'un ou plusieurs alcènes comprennent l'éthylène et le propylène et l'un ou plusieurs alcanes comprennent l'éthane et le propane.

4. Procédé selon l'une quelconque des revendications précédentes, dans lequel le mélange peut être obtenu par craquage catalytique du pétrole.

5. Procédé selon la revendication 1 ou 2, dans lequel l'un ou plusieurs alcènes comprennent l'éthylène et le propylène et l'un ou plusieurs alcanes comprennent le méthane, l'éthane et le propane.

6. Procédé selon l'une quelconque des revendications 1 à 3 et 5, dans lequel le mélange comprend l'hydrogène, le monoxyde de carbone, le méthane, l'éthylène, l'éthane, le propylène et le propane, éventuellement dans lequel le mélange comprend en outre un gaz inerte, éventuellement dans lequel le gaz inerte comprend l'azote.

7. Procédé selon l'une quelconque des revendications 1 à 3, 5 et 6 dans lequel le mélange peut être obtenu par craquage thermique du pétrole.

8. Procédé selon l'une quelconque des revendications 1 à 3, 5 et 6, dans lequel le mélange est du gaz naturel, du gaz de schiste, du gaz de houille, du biogaz, du gaz de raffinerie ou un gaz pouvant être obtenu par craquage catalytique et/ou thermique du pétrole.

9. Procédé selon l'une quelconque des revendications précédentes, dans lequel le mélange comprend en outre un ou plusieurs composés organosoufrés, la première phase comprend en outre l'un ou plusieurs composés organosoufrés, et la deuxième phase a une teneur réduite de l'un ou plusieurs composés organosoufrés.

10. Procédé selon l'une quelconque des revendications précédentes, dans lequel l'agent d'extraction comprend ledit méthanol en une quantité égale ou supérieure à 50 % en poids.

11. Procédé selon l'une quelconque des revendications précédentes, dans lequel l'agent d'extraction comprend ledit méthanol qui est un premier solvant polaire, et un deuxième solvant qui est différent dudit premier solvant polaire et est miscible avec le premier solvant polaire, dans lequel le deuxième solvant est un deuxième solvant polaire, et dans lequel les premier et deuxième solvants polaires sont des solvants organiques polaires différents, et dans lequel chacun des premier et deuxième solvants a un paramètre de solubilité Hildebrand ($\delta$) qui est égal ou supérieur à 18 MPa$^{1/2}$ et un indice de polarité égal ou supérieur à 3.

12. Procédé selon l'une quelconque des revendications précédentes, dans lequel l'agent d'extraction comprend ledit méthanol et ledit carbonate de diméthyle (DMC).

13. Procédé selon l'une quelconque des revendications précédentes, dans lequel la mise en contact est réalisée :

   à une première température, laquelle première température est de -30°C à 60°C et éventuellement de 5°C à 35°C ; et
   à une première pression, laquelle première pression est supérieure à 1 bar et éventuellement supérieure à 1 bar et inférieure ou égale à 300 bars.

14. Procédé selon l'une quelconque des revendications précédentes qui comprend en outre la récupération du mélange ayant une teneur réduite de l'un ou plusieurs premiers hydrocarbures, éventuellement dans lequel la récupération dudit mélange ayant une teneur réduite de l'un ou plusieurs premiers hydrocarbures comprend la séparation de la deuxième phase de la première phase.

15. Procédé selon l'une quelconque des revendications précédentes qui comprend en outre la récupération de l'un ou plusieurs premiers hydrocarbures de la première phase dans lequel, après la récupération de l'un ou plusieurs premiers hydrocarbures de la première phase, le procédé comprend éventuellement en outre la mise en contact de la deuxième phase avec l'agent d'extraction de la première phase, et formant ainsi :

   (i) une autre première phase comprenant l'agent d'extraction et lesdits un ou plusieurs premiers hydrocarbures ; et
   (ii) une autre deuxième phase comprenant ledit mélange ayant une teneur encore réduite de l'un ou plusieurs premiers hydrocarbures ;
   éventuellement dans lequel le procédé comprend en outre :

      (a) la récupération de l'un ou plusieurs premiers hydrocarbures de l'autre première phase, dans lequel, après la récupération de l'un ou plusieurs premiers hydrocarbures de l'autre première phase, le procédé comprend éventuellement en outre :

(b) la mise en contact de l'autre deuxième phase avec l'agent d'extraction de l'autre première phase, et formant ainsi : (i) une autre première phase comprenant l'agent d'extraction et lesdits un ou plusieurs premiers hydrocarbures, et (ii) une autre deuxième phase comprenant ledit mélange ayant une teneur encore réduite de l'un ou plusieurs premiers hydrocarbures ; et

(c) la répétition éventuelle des étapes (a) et (b) une ou plusieurs fois.

**Fig. 1**

EP 3 377 598 B1

**Fig. 2**

**Fig. 3**

| Sample No. | Solvent (1:1 in volume) | Polarity Index of Added Solvent | Hildebrand Parameter ($\delta$) of Added Solvent | Hansen Parameters of Added solvent | | | $S_i$ Paraffin/Olefin ratio (adsorption) | $S_i$ Paraffin/Olefin ratio (desorption) |
|---|---|---|---|---|---|---|---|---|
| | | | | $\delta_d$ | $\delta_p$ | $\delta_h$ | | |
| | | | | C2 Content | | | | |
| i | n-hexane+DMC | 0.1 | 14.9 | 14.9 | 0.0 | 0.0 | 1.47 | 2.12 |
| ii | pure DMC | 3.9 | 20.6 | 15.8 | 7.5 | 10.9 | 2.11 | 1.52 |
| iii | methanol+DMC | 5.1 | 29.7 | 15.1 | 12.3 | 22.3 | 2.25 | 1.52 |
| iv | Propylene Carbonate+DMC | 6.1 | 27.2 | 20.0 | 18.0 | 4.1 | 2.33 | 1.41 |
| v | DMSO+DMC | 7.2 | 24.5 | 18.4 | 16.4 | 10.2 | 2.46 | 1.21 |
| | | | | C3 Content | | | | |
| i | n-hexane+DMC | 0.1 | 14.9 | 14.9 | 0.0 | 0.0 | 2.42 | 2.02 |
| ii | pure DMC | 3.9 | 20.6 | 15.8 | 7.5 | 10.9 | 3.65 | 1.99 |
| iii | methanol+DMC | 5.1 | 29.7 | 15.1 | 12.3 | 22.3 | 4.13 | 1.90 |
| iv | Propylene Carbonate+DMC | 6.1 | 27.2 | 20.0 | 18.0 | 4.1 | 5.60 | 1.78 |
| v | DMSO+DMC | 7.2 | 24.5 | 18.4 | 16.4 | 10.2 | 7.13 | 1.52 |

**Fig. 4**

**Fig. 5**

**Fig. 6**

| Sample No. | Solvent (1:1 in volume) | Polarity Index of Added Solvent | Hildebrand Parameter ($\delta$) of Added Solvent | Hansen Parameters of Added solvent | | | Total Adsorbed Gas Volume (ml) |
|---|---|---|---|---|---|---|---|
| | | | | $\delta_d$ | $\delta_p$ | $\delta_h$ | |
| i | n-hexane+DMC | 0.1 | 14.9 | 14.9 | 0.0 | 0.0 | 6630 |
| ii | pure DMC | 3.9 | 20.6 | 15.8 | 7.5 | 10.9 | 3570 |
| iii | methanol+DMC | 5.1 | 29.7 | 15.1 | 12.3 | 22.3 | 3525 |
| iv | Propylene Carbonate+DMC | 6.1 | 27.2 | 20.0 | 18.0 | 4.1 | 1300 |
| v | DMSO+DMC | 7.2 | 24.5 | 18.4 | 16.4 | 10.2 | 1073 |

**Fig. 7**

**Fig. 8**

| Sample No. | Solvent (1:1 in volume) | Polarity Index of Added Solvent | Hildebrand Parameter ($\delta$) of Added Solvent | Hansen Parameters of Added solvent | | | $S_i$ Paraffin/Olefin ratio (adsorption) | $S_i$ Paraffin/Olefin ratio (desorption) |
|---|---|---|---|---|---|---|---|---|
| | | | | $\delta_d$ | $\delta_p$ | $\delta_h$ | | |
| | | | | C2 Content | | | | |
| vi | n-hexane+DMC | 0.1 | 14.9 | 14.9 | 0.0 | 0.0 | 0.25 | 0.12 |
| vii | pure DMC | 3.9 | 20.6 | 15.8 | 7.5 | 10.9 | 0.12 | 0.09 |
| viii | methanol+DMC | 5.1 | 29.7 | 15.1 | 12.3 | 22.3 | 0.14 | 0.09 |
| ix | Propylene Carbonate+DMC | 6.1 | 27.2 | 20.0 | 18.0 | 4.1 | 0.16 | 0.08 |
| x | DMSO+DMC | 7.2 | 24.5 | 18.4 | 16.4 | 10.2 | 0.17 | 0.08 |
| | | | | C3 Content | | | | |
| vi | n-hexane+DMC | 0.1 | 14.9 | 14.9 | 0.0 | 0.0 | 1.92 | 0.82 |
| vii | pure DMC | 3.9 | 20.6 | 15.8 | 7.5 | 10.9 | 0.47 | 0.26 |
| viii | methanol+DMC | 5.1 | 29.7 | 15.1 | 12.3 | 22.3 | 0.49 | 0.18 |
| ix | Propylene Carbonate+DMC | 6.1 | 27.2 | 20.0 | 18.0 | 4.1 | 0.56 | 0.17 |
| x | DMSO+DMC | 7.2 | 24.5 | 18.4 | 16.4 | 10.2 | 0.67 | 0.16 |

**Fig. 9**

**Fig. 10**

**Fig. 11**

| Sample No. | Solvent (1:1 in volume) | Polarity Index of Added Solvent | Hildebrand Parameter ($\delta$) of Added Solvent | Hansen Parameters of Added solvent | | | Total Adsorbed Gas Volume (ml) |
|---|---|---|---|---|---|---|---|
| | | | | $\delta_d$ | $\delta_p$ | $\delta_h$ | |
| vi | n-hexane+DMC | 0.1 | 14.9 | 14.9 | 0.0 | 0.0 | 680 |
| vii | pure DMC | 3.9 | 20.6 | 15.8 | 7.5 | 10.9 | 645 |
| x | DMSO+DMC | 7.2 | 24.5 | 18.4 | 16.4 | 10.2 | 620 |
| ix | Propylene Carbonate+DMC | 6.1 | 27.2 | 20.0 | 18.0 | 4.1 | 615 |
| viii | methanol+DMC | 5.1 | 29.7 | 15.1 | 12.3 | 22.3 | 530 |

**Fig. 12**

**Fig. 13**

| Gas Content | MTCGM (mol%) | n-hexane+DMC (mol%) (vi) | pure DMC (mol%) (vii) | methanol+DMC (mol%) (viii) | propylene carbonate+DMC (mol%) (ix) | DMSO+DMC (mol%) (x) |
|---|---|---|---|---|---|---|
| $H_2$ Fraction | 15.00% | 0.71% | 0.76% | 1.10% | 0.74% | 0.16% |
| CO Fraction | 4.01% | 1.00% | 0.89% | 1.23% | 0.67% | 0.14% |
| $CH_4$ Fraction | 24.76% | 23.56% | 16.38% | 15.16% | 11.00% | 6.14% |

**Fig. 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3527837 A
- US 5085741 A

**Non-patent literature cited in the description**

- **ELDRIDGE, R. B.** *Industrial & engineering chemistry research,* 1993, vol. 32, 2208
- **RUTHVEN, D. M. ; REYES, S. C.** *Microporous and mesoporous materials,* 2007, vol. 104, 59
- **CORMA, A. ; MELO, F. ; SAUVANAUD, L. ; ORTEGA, F.** *Catalysis today,* 2005, vol. 107, 699
- **BAKER, L. M. ; CARRICK, W. L.** *The Journal of Organic Chemistry,* 1970, vol. 35, 774
- **KILTY, P. ; SACHTLER, W.** *Catalysis Reviews Science and Engineering,* 1974, vol. 10, 1
- **DU, Y. ; WANG, H. ; CHEN, S.** *Journal of Molecular Catalysis A: Chemical,* 2002, vol. 179, 253
- **ORTIZ, A. ; RUIZ, A. ; GORRI, D. ; ORTIZ, I.** *Separation and Purification Technology,* 2008, vol. 63, 311
- **REINE, T. A. ; ELDRIDGE, R. B.** *Industrial & engineering chemistry research,* 2005, vol. 44, 7505
- **SAFARIK, D. J. ; ELDRIDGE, R. B.** *Industrial & Engineering Chemistry Research,* 1998, vol. 37, 2571
- **YANG, R. ; KIKKINIDES, E.** *AIChE Journal,* 1995, vol. 41, 509
- **ORTIZ, A. ; MARIA GALÁN, L. ; GORRI, D. ; DE HAAN, A. B. ; ORTIZ, I.** *Industrial & Engineering Chemistry Research,* 2010, vol. 49, 7227
- **KRULL, F. ; MEDVED, M. ; MELIN, T.** *Chemical Engineering Science,* 2007, vol. 62, 5579
- **BRYAN, R.** *Sep. Purif. ReV.,* 2004, vol. 33, 157
- **KELLER, G. E. ; MARCINKOWSKY, A. E. ; VERMA, S. K. ; WILLIAMSON, K. D.** Olefin recovery and purification via silver complexation. Marcel Dekker, 1992
- **SHU, C. ; KULVARANON, S. ; FINDLEY, M. ; LIAPIS, A.** *Separations Technology,* 1990, vol. 1, 18
- **KULVARANON, S. ; FINDLEY, M. E. ; LIAPIS, A. I.** *Industrial & engineering chemistry research,* 1990, vol. 29, 106
- **SCHOELLNER, R. ; MUELLER, U.** *Adsorption science & technology,* 1986, vol. 3, 167
- **TAJBL, D. ; KANOFSKY, J. ; BRABAND, J.** *Energy Process./Can.,* 1980, vol. 72 (5), 61
- **FAIZ, R. ; LI, K.** *Desalination,* 2012, vol. 287, 82
- **HO, W. W. ; DOYLE, G. ; SAVAGE, D. W. ; PRUETT, R. L.** *Industrial & engineering chemistry research,* 1988, vol. 27, 334
- **HUGHES, R. ; MAHONEY, J. ; STEIGELMANN, E.** *Recent Developments in Separation Science,* 1986, vol. 9, 173
- **A F BARTON.** *Chemical Reviews,* 1975, vol. 75 (6), 731
- **BARTON, A. F.** CRC handbook of solubility parameters and other cohesion parameters. CRC press, 1991
- **REICHARDT, C. ; WELTON, T.** Solvents and solvent effects in organic chemistry. John Wiley & Sons, 2011
- **L R SNYDER.** *J of Chromatography,* 1974, vol. 92, 223
- **YEO, G. A. ; FORD, T. A.** *Canadian journal of chemistry,* 1991, vol. 69, 632
- **DESIRAJU, G. R. ; STEINER, T.** The weak hydrogen bond: in structural chemistry and biology. Oxford University Press, 2001
- **MÜLLER, E. A. ; GUBBINS, K. E.** *Industrial & Engineering Chemistry Research,* 2001, vol. 40, 2193
- **LEE, L.-H.** Fundamentals of adhesion. Springer, 1991
- **GERALD, R. ; BERNHARD, T. ; HAEBERLEN, U. ; RENDELL, J. ; OPELLA, S.** *Journal of the American Chemical Society,* 1993, vol. 115, 777
- **VAN MOURIK, T. ; VAN DUIJNEVELDT, F. B.** *Journal of Molecular Structure: THEOCHEM,* 1995, vol. 341, 63
- **PRAUSNITZ, J. ; ANDERSON, R.** *AIChE Journal,* 1961, vol. 7, 96
- **BRINCK, T. ; MURRAY, J. S. ; POLITZER, P.** *Molecular Physics,* 1992, vol. 76, 609
- **PRITCHARD, H. ; SKINNER, H.** *Chemical Reviews,* 1955, vol. 55, 745
- **SNYDER, L.** *Journal of Chromatographic Science,* 1978, vol. 16, 223

- **SNYDER, L. R. ; KIRKLAND, J. J. ; GLAJCH, J. L.** Practical HPLC method development. John Wiley & Sons, 2012
- **MACLAY, W. N.** *Journal of Colloid Science,* 1956, vol. 11, 272
- **AHUJA, S.** Chromatography and Separation Science. Academic Press, 2003
- **LEI, Z. ; LI, C. ; CHEN, B.** *Separation & Purification Reviews,* 2003, vol. 32, 121
- **KYLE, B. ; LENG, D.** *Industrial & Engineering Chemistry,* 1965, vol. 57, 43
- **BELMARES, M. ; BLANCO, M. ; GODDARD, W. ; ROSS, R. ; CALDWELL, G. ; CHOU, S. H. ; PHAM, J. ; OLOFSON, P. ; THOMAS, C.** *Journal of computational chemistry,* 2004, vol. 25, 1814
- **HANSEN, C. M.** *Danish Technical: Copenhagen,* 1967, 14
- **HANSEN, C. M.** Hansen solubility parameters: a user's handbook. CRC press, 2012
- **HANSEN, C. M.** *Progress in Organic Coatings,* 2004, vol. 51, 77
- **LEWIS, W. ; WHITMAN, W.** *Industrial & Engineering Chemistry,* 1924, vol. 16, 1215
- **BATTINO, R. ; CLEVER, H. L.** *Chemical Reviews,* 1966, vol. 66, 395
- **FROLICH, P. K. ; TAUCH, E. J. ; HOGAN, J. J. ; PEER, A. A.** *Industrial & Engineering Chemistry,* 1931, vol. 23, 548
- Gas Market Research Report of China. China National Chemical Information Center, 2012
- *Gas Market Research Report of China,* 2011